(19) Europäisches Patentamt
European Patent Office
Office européen des brevets

(11) **EP 2 796 105 B1**

(12) **EUROPEAN PATENT SPECIFICATION**

(45) Date of publication and mention
of the grant of the patent:
**01.11.2017 Bulletin 2017/44**

(51) Int Cl.:
*A61B 18/12* *(2006.01)*     *A61B 17/32* *(2006.01)*
*A61B 18/14* *(2006.01)*

(21) Application number: **13781989.2**

(22) Date of filing: **16.04.2013**

(86) International application number:
**PCT/JP2013/061266**

(87) International publication number:
**WO 2013/161624 (31.10.2013 Gazette 2013/44)**

(54) **SURGICAL SYSTEM**

CHIRURGISCHES SYSTEM

SYSTÈME CHIRURGICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.04.2012 US 201261638741 P**

(43) Date of publication of application:
**29.10.2014 Bulletin 2014/44**

(73) Proprietor: **Olympus Corporation
Tokyo 192-8507 (JP)**

(72) Inventors:
• **KABAYA, Akinori**
**Hachioji-shi,**
**Tokyo 192-8507 (JP)**
• **SHIMIZU, Ko**
**Hachioji-shi,**
**Tokyo 192-8507 (JP)**

• **NAKABE, Kazuya**
**Hachioji-shi,**
**Tokyo 192-8507 (JP)**
• **IRISAWA, Takashi**
**Hachioji-shi,**
**Tokyo 192-8507 (JP)**

(74) Representative: **Gunzelmann, Rainer**
**Wuesthoff & Wuesthoff**
**Patentanwälte PartG mbB**
**Schweigerstraße 2**
**81541 München (DE)**

(56) References cited:
**WO-A1-2011/089769**     **JP-A- H11 267 130**
**JP-A- 2002 078 715**     **JP-A- 2005 102 811**
**JP-A- 2005 102 811**     **US-A1- 2011 015 627**
**US-A1- 2012 078 139**

## Description

Technical Field

The present invention relates to a surgical operation system that performs a surgical operation with use of high frequency energy and other energy.

Background Art

Conventionally, treatment such as resection and dissection by supplying high frequency energy to a living tissue of an object to be treated has been widely performed.

In recent years, treatment such as resection and dissection has been performed by also supplying ultrasound energy to the living tissue of an object to be treated, in addition to high frequency energy.

For example, the conventional example of U. S. Patent Publication No. 2008/0132887 discloses a surgical operation apparatus that grasps a living tissue with a strong grasping force by using high frequency current and ultrasound vibration, and performs coagulation/dissection.

Document WO 2011/089769 A1 also discloses a surgical apparatus which combines ultrasonic vibration and high frequency current to treat biological tissue. If wire breakage occurs to a high frequency signal line that transmits a high frequency signal when treatment is performed with use of high frequency energy and other energy such as ultrasound energy, it is desired to be able to cope with the occurrence of wire breakage quickly, but the above described conventional example does not teach the substance of detection of occurrence of wire breakage.

In a case of performing the treatment only by the high frequency energy, if the wire breakage occurs, the treatment cannot be performed at all and therefore the user easily recognizes the occurrence of the wire breakage.

However, in a case of performing the treatment by using the high frequency energy and also the other energy, it is difficult to promptly recognize the occurrence of wire breakage since the treatment can be performed by the other energy. Further, in this case, since the situation is changed from a state of performing the treatment using both of the energies into a state where the function is lowered, there is a possibility of taking time for performing the treatment since the treatment is performed in the state where treatment ability is lowered by only one of the energies whereas appropriate treatment can be completed in a short time in the state where the both energies are used.

The present invention has been made in view of the foregoing points and an object of the present invention is to provide a surgical operation system capable of immediately detecting occurrence of breakage of a high frequency signal wire and performing control of stopping an output to promptly cope with the wire breakage in a case of performing treatment using both of a high frequency energy and other energy.

Disclosure of Invention

The invention is defined in claim 1.

Brief Description of the Drawings

Fig. 1 is a view showing an entire configuration of a surgical operation system of a first embodiment of the present invention;

Fig. 2 is a diagram showing an internal configuration of a power supply apparatus that configures the surgical operation system;

Fig. 3 is a diagram showing an equivalent circuit of a high frequency signal line of a treatment instrument including a cable that is connected to a high frequency output terminal of the power supply apparatus;

Fig. 4 is a diagram showing outlines of waveforms of a high frequency voltage and a high frequency current that are supplied from the high frequency output terminal to a treatment instrument side;

Fig. 5 is a diagram showing the high frequency voltage and the high frequency current that are supplied from the high frequency output terminal to the treatment instrument side by vectors;

Fig. 6 is a flowchart showing a processing content in a case in which treatment is performed that includes a processing content of detecting occurrence of wire breakage in the first embodiment;

Fig. 7A is an explanatory diagram showing that an electrostatic capacity changes when wire breakage does not occur to the high frequency signal line of the treatment instrument including the cable and when wire breakage occurs;

Fig. 7B is a flowchart showing a processing content of a modification in the first embodiment;

Fig. 8 is a view showing a surgical operation system of a second embodiment of the present invention;

Fig. 9 is a diagram showing an internal configuration of a power supply apparatus in the second embodiment;

Fig. 10 is a flowchart showing a processing content in a case in which treatment is performed that includes a processing content of detecting occurrence of wire breakage in the second embodiment;

Fig. 11 is a flowchart showing a processing content in a case in which treatment is performed that includes a processing content of detecting occurrence of wire breakage in a third embodiment of the present invention;

Fig. 12 is a flowchart showing a processing content of detecting occurrence of wire breakage in a fourth embodiment of the present invention;

Fig. 13 is a flowchart showing a processing content of detecting occurrence of wire breakage in a first modification of the fourth embodiment;

Fig. 14 is a diagram showing an internal configuration of a power supply apparatus in a second modification of the fourth embodiment;

Fig. 15 is a diagram showing impedance values that are measured when a frequency is changed, in a case in which electrostatic capacity values of the treatment instrument are different values;

Fig. 16 is a flowchart showing a processing content of detecting occurrence of wire breakage in the second modification of the fourth embodiment;

Fig. 17 is a flowchart showing a processing content of detecting occurrence of wire breakage with a processing procedure different from that of Fig. 16; and

Fig. 18 is a flowchart showing a processing content of detecting occurrence of wire breakage in a third modification of the fourth embodiment.

Best Mode for Carrying Out the Invention

Hereinafter, respective embodiments of the present invention will be described with reference to the drawings.

(First Embodiment)

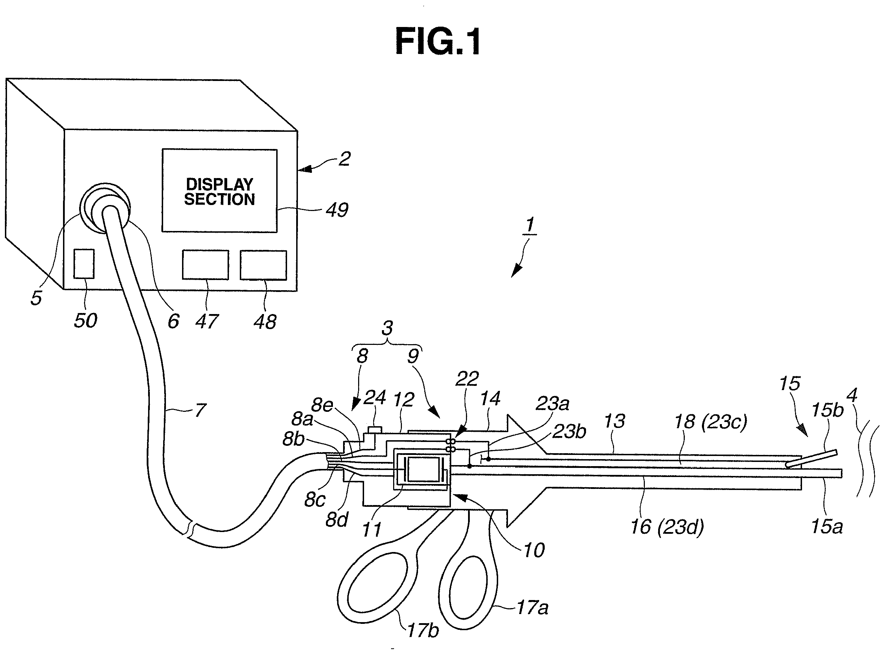

As shown in Fig. 1, a surgical operation system 1 of a first embodiment of the present invention is configured by a power supply apparatus 2 capable of simultaneously outputting a high frequency signal as a first signal for supplying high frequency energy to a living tissue, and a second signal for supplying other energy different from the high frequency energy to the living tissue, and a high frequency/ultrasound treatment instrument (hereinafter, abbreviated simply as a treatment instrument) 3 as a surgical treatment instrument that is detachably connected to the power supply apparatus 2, and performs treatment such as sealing to a blood vessel 4, for example, as a living tissue of an object to be treated by high frequency energy and ultrasound energy. Note that in the present embodiment, the second signal is an ultrasound drive signal, because as the other energy different from high frequency energy, ultrasound energy, for example, is adopted.

The treatment instrument 3 is configured by an ultrasound transducer unit 8 including a cable 7 having a connector 6 that is detachably connected to a connector receiver 5 of the power supply apparatus 2 provided at one end portion, and a handle unit 9 including a fitting portion 10 to which a front end of the ultrasound transducer unit 8 is detachably fitted.

The ultrasound transducer unit 8 has a main body unit 12 containing an ultrasound transducer 11 as a converter that converts an ultrasound drive signal into ultrasound (ultrasound vibration) that forms ultrasound energy by the ultrasound drive signal being applied (supplied) thereto, and a front end side of the main body unit 12 is fitted to the fitting portion 10 at a proximal end side of the handle unit 9. The cable 7 is extended from a rear end of the main body unit 12.

The handle unit 9 has an elongated insertion portion (or a sheath portion) 13, and an operation portion 14 provided at a proximal end (a rear end) of the insertion portion 13, and a treatment portion 15 that performs treatment by using high frequency energy and ultrasound energy is provided at a distal end of the insertion portion 13.

A probe 16 that is formed by an ultrasound transmitting rod that transmits ultrasound vibration by the ultrasound transducer 11 is inserted through an inside of the insertion portion 13, and a distal end of the probe 16 is a grasping member 15a at a fixed side (of a pair of grasping members 15a and 15b) that configures the treatment portion 15.

Further, the operation portion 14 is provided with finger rests 17a and 17b for performing an operation of opening and closing the grasping members 15a and 15b. A surgeon lays his or her fingers on the finger rests 17a and 17b, and performs an operation of opening and closing the finger rest 17b with respect to the finger rest 17a, whereby the surgeon pulls a wire 18 that is inserted through the inside of the insertion portion 13 to open and close the grasping member 15b at a movable side with respect to the grasping member 15a, and can grasp a living tissue of an object to be treated such as the blood vessel 4, and stop grasping. Note that a distal end of the wire 18 is fixed to the grasping member 15b, and a rear end of the wire 18 moves in a longitudinal direction of the insertion portion 13 by being linked to an operation of the finger rest 17b. Further, the grasping member 15b at the movable side is also called a jaw.

Further, in the present embodiment, the above described grasping members 15a and 15b have a function of a bipolar electrode that configures a pair of electrodes that perform treatment to a living body by passing a high frequency current.

Therefore, the high frequency signal that is outputted from the high frequency output terminal of the power supply apparatus 2 is supplied to the grasping members 15a and 15b that function as a bipolar electrode via high frequency signal lines 8a and 8b inside the ultrasound transducer unit 8 including the cable 7, high frequency signal lines 23a and 23b inside the handle unit 9 that electrically continue respectively to the high frequency signal lines 8a and 8b in contact point portions 22, and the wire 18 and the conductive probe 16 to which the high frequency signal lines 23a and 23b are respectively connected. Note that the high frequency signal line 23a is connected to a vicinity of the rear end of the wire 18, and the high frequency signal line 23b is connected to a vicinity of a rear end of the probe 16.

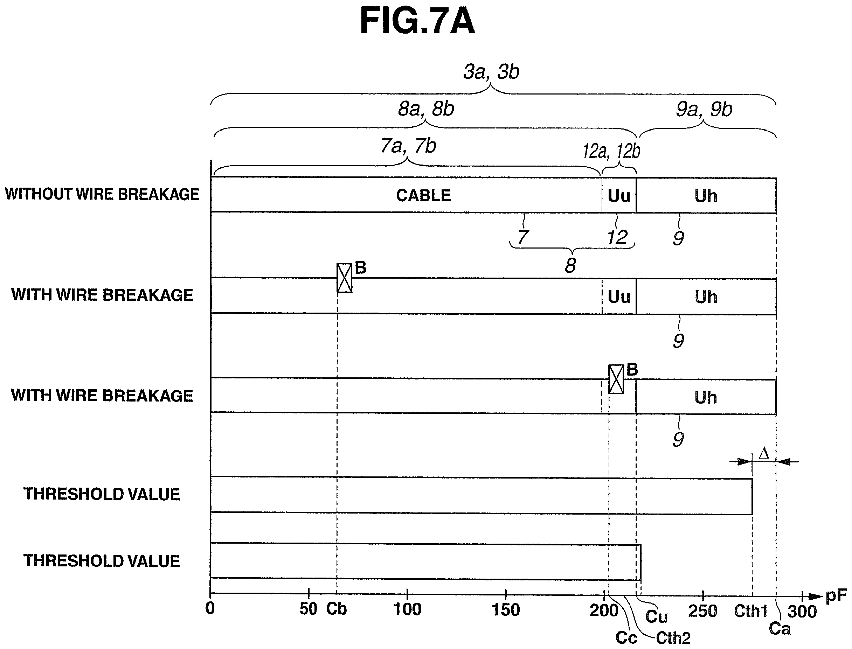

As above, the probe 16 and the wire 18 are formed from conductors of a metal or the like that transmit the high frequency signal. A configuration may be adopted, that connects the high frequency signal line 23a to the grasping member 15a by inserting the high frequency signal line 23a through the inside of the insertion portion 13 instead of connecting the high frequency signal line 23a to the rear end side of the wire 18. Further, a configuration may be adopted that connects the high frequency signal line 23b to the grasping member 15b (one of the electrodes that configure the bipolar electrode) by inserting the high frequency signal line 23b through the inside of the insertion portion 13 without connecting the high frequency signal line 23b to the probe 16. Subsequently, a high frequency current is passed to the living tissue grasped by the grasping members 15a and 15b that configure the treatment portion 15, whereby treatment by high frequency energy can be performed. Note that in the following description, the high frequency signal lines 23a and 23b inside the handle unit 9, a high frequency signal line 23c by the wire 18, and a high frequency signal line 23d by the probe 16 will be brought together and expressed by high frequency signal lines 9a and 9b as shown in Fig. 7A.

Further, the high frequency signal lines 8a and 8b that are inserted through the inside of the ultrasound transducer unit 8 including the cable 7 are configured by high frequency signal lines 7a and 7b inside the cable 7 and high frequency signal lines 12a and 12b inside the main body unit 12, as shown in Fig. 7A. Note that in Fig. 7A, the main body unit 12 in a case in which the ultrasound transducer unit 8 is divided into the cable 7 and the main body unit 12 is simply shown by Uu, and the handle unit 9 is simply shown by Uh.

As is understood from Fig. 1, as compared with lengths of the high frequency signal lines 7a and 7b in the cable 7, lengths of the high frequency signal lines 12a and 12b in the main body unit 12 are sufficiently shorter (normally, 1/10 of the former or less), and therefore, an electrostatic capacity between the high frequency signal lines 12a and 12b of the latter (the high frequency signal lines 12a and 12b) with respect to the former (the high frequency signal lines 7a and 7b) is sufficiently small (normally, an electrostatic capacity of 1/10 of that of the former or less).

Further, to the ultrasound transducer 11 inside the above described main body unit 12, an ultrasound drive signal is applied from the output terminal for ultrasound of the power supply apparatus 2 via signal lines 8c and 8d (for ultrasound) inside the cable 7 and inside the main body unit 12.

Further, on an outer circumferential face of an upper side of the main body unit 12, an output switch 24 that performs an instruction operation of output/stop of output (ON/OFF) of a high frequency signal and an ultrasound drive signal is provided. The ON/OFF signal by an operation of the output switch 24 is outputted to a control section 42 in an inside of the power supply apparatus 2 via a signal line 8e inside the main body unit 12 and the cable 7.

Note that while the ultrasound transducer unit 8 in the present embodiment is a reuse product that is repeatedly used by being cleaned and sterilized, the handle unit 9 is a disposable product that is thrown away at each surgical operation for one case.

In other words, every time one surgical operation is performed, the treatment instrument 3 with use of the new handle unit 9 that is replaced and used and the ultrasound transducer unit 8 that is repeatedly used is used.

In this case, the new handle unit 9 is guaranteed to have the high frequency signal lines 9a and 9b inside the handle unit 9 without wire breakage. Further, in ordinary cases, the handle unit 9 has such high reliability that in a use time period of one surgical operation, the high frequency signal lines 9a and 9b inside the handle unit 9 do not have wire breakage.

In contrast with the above, the ultrasound transducer unit 8 to which the cable 7 is integrally connected is repeatedly used for a long period of time, and therefore, as compared with the case of the handle unit 9, the high frequency signal lines 7a and 7b inside the cable 7 or the high frequency signal lines 12a and 12b inside the main body unit 12 are likely to have wire breakage. Therefore, in the present embodiment, a wire breakage detecting section that detects occurrence of wire breakage is provided inside the power supply apparatus 2 as will be described as follows.

Note that when wire breakage occurs in an apparatus that performs treatment with only high frequency energy, a surgeon can easily recognize occurrence of the wire breakage, because the high frequency energy is not outputted to a living tissue, and the surgeon cannot proceed with the treatment.

In contrast with the above, during the process of performing treatment by supplying high frequency energy and ultrasound energy as the other energy to a living tissue of an object to be treated, the treatment by only the ultrasound energy can be performed even if wire breakage occurs, and therefore, the surgeon sometimes cannot immediately recognize the time when the wire breakage occurs.

In this case, the treatment function is changed to a smaller treatment function as compared with the treatment function of treating with use of both kinds of energy that are the high-frequency energy and the ultrasound energy.

Therefore, when treatment is performed with use of both kinds of energy that are the high frequency energy and the ultrasound energy as above, it is desirable to stop treatment with the treatment instrument where wire breakage occurs, and to assist so as to notify the surgeon of occurrence of the wire breakage quickly.

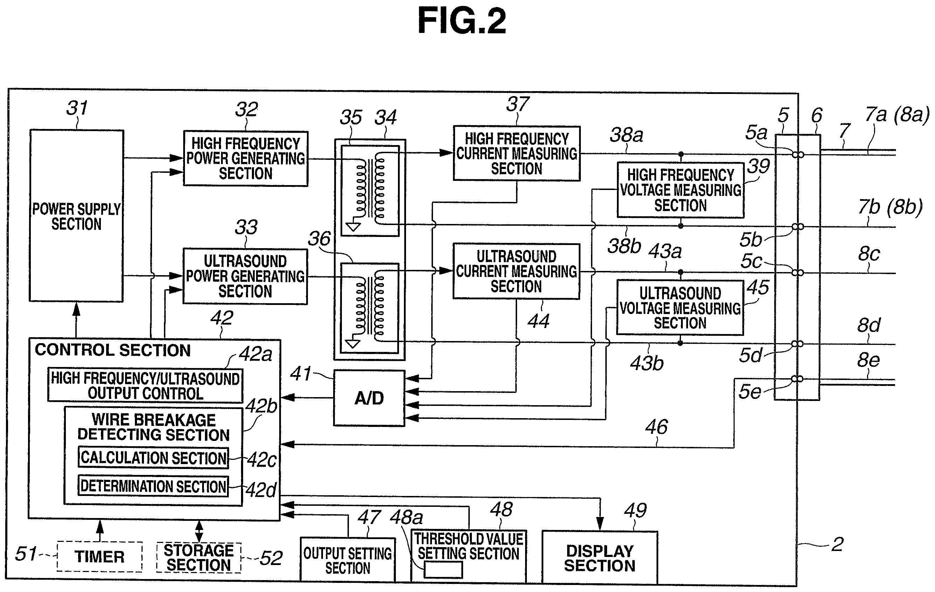

Fig. 2 shows an internal configuration of the power supply apparatus 2 including a function of detecting occurrence of wire breakage.

A power supply section 31 inside the power supply apparatus 2 generates a direct-current power from an alternating-current power, and supplies the generated direct-current power to a high frequency power generating section 32 as a first signal generating section, and an ultrasound drive power generating section (abbreviated as an ultrasound power generating section) 33 as a second signal generating section.

The high frequency power generating section 32 generates high frequency power from direct-current power, and outputs the high frequency power to a primary side of a first output transformer 35 that configures an output transformer 34.

The ultrasound power generating section 33 generates ultrasound drive power from direct-current power, and outputs the ultrasound drive power to a primary side of a second output transformer 36 that configures the output transformer 34.

At a secondary side of the first output transformer 35, high frequency power that is insulated from the primary side thereof is induced (generated). One terminal in two terminals at the secondary side of the first output transformer 35 is connected to a contact point 5a of the connector receiver 5 via a high frequency current measuring section (or a high frequency current detecting section) 37 that is provided halfway through a high frequency signal line 38a and measures (or detects) a high frequency current, and the other terminal is connected to a contact point 5b of the connector receiver 5 via a high frequency signal line 38b, respectively.

The contact points 5a and 5b of the connector receiver 5 configure the high frequency output terminal, and the high frequency signal as the first signal that is outputted from the contact points 5a and 5b is supplied to the grasping members 15a and 15b that perform the function of the bipolar electrode of the treatment portion 15 via the high frequency signal lines 8a and 8b (namely, the high frequency signal lines 7a and 7b inside the cable 7, and the high frequency signal lines 12a and 12b inside the main body unit 12) of the ultrasound transducer unit 8, and the high frequency signal lines 9a and 9b of the handle unit 9.

The high frequency signal lines 38a and 38b in the power supply apparatus 2 are connected to a high frequency voltage measuring section (or a high frequency voltage detecting section) 39, and the high frequency voltage measuring section 39 measures (or detects) the high frequency voltage at the secondary side of the first output transformer 35.

The high frequency current that is measured by the above described high frequency current measuring section 37, and the high frequency voltage that is measured by the high frequency voltage measuring section 39 are inputted into the control section 42 that controls operations of respective sections of the power supply apparatus 2 via an A/D converter 41.

Further, at a secondary side of the second output transformer 36, ultrasound drive power that is insulated from the primary side thereof is induced (generated). One terminal in two terminals at the secondary side of the second output transformer 36 is connected to a contact point 5c of the connector receiver 5 via an ultrasound current measuring section (or an ultrasound current detecting section) 44 that is provided halfway through an ultrasound drive signal line (also simply described as a signal line) 43a and measures (or detects) an ultrasound drive current, and the other terminal is connected to a contact point 5d of the connector receiver 5 via a signal line 43b respectively.

The contact points 5c and 5d of the connector receiver 5 configure the output terminal for ultrasound, and the ultrasound drive signal as the second signal that is outputted from the contact points 5c and 5d is supplied (applied) to the ultrasound transducer 11 as a converter via the signal lines 8d and 8d inside the ultrasound transducer unit 8. Subsequently, the ultrasound transducer 11 performs ultrasound vibration by application of the ultrasound drive signal, and the ultrasound vibration is transmitted by the probe 16 to the grasping member 15a at the distal end thereof.

The signal lines 43a and 43b inside the power supply apparatus 2 are connected to an ultrasound voltage measuring section 45, and the ultrasound voltage measuring section (or an ultrasound voltage detecting section) 45 measures (or detects) the ultrasound drive voltage at the secondary side of the second output transformer 36.

The ultrasound drive current that is measured by the above described ultrasound current measuring section 44 and the ultrasound drive voltage that is measured by the ultrasound voltage measuring section 45 are inputted into the control section 42 via the A/D converter 41.

Further, the ON/OFF instruction signal of the output switch 24 is applied to a contact point 5e of the connector receiver 5 via the signal line 8e. Subsequently, the ON/OFF signal is inputted into the control section 42 via a signal line 46.

The control section 42 simultaneously controls generation/stop of generation of the high frequency power by the high frequency power generating section 32, and generation/stop of generation of the ultrasound drive power by the ultrasound power generating section 33, in accordance with the ON/OFF instruction signal of the output switch 24. In other words, the control section 42 performs control of supply/stop of supply of the high frequency power and the ultrasound drive power, with respect to the treatment instrument 3 from the power supply apparatus 2, in accordance with the ON/OFF instruction signal of the output switch 24.

The control section 42 has a function of a high frequency/ultrasound output control section 42a that performs control of supply/stop of supply of high frequency power and ultrasound drive power in accordance with the ON/OFF instruction signal from the output switch 24, and performs output control of a high frequency signal and an ultrasound drive signal in response to output setting of an output setting section 47.

The control section 42 calculates a resistance component of a living tissue from the high frequency voltage measured by the high frequency voltage measuring section 39, and the high frequency current measured by the high frequency current measuring section 37, and performs output control of the high frequency signal in a case in which the living tissue is treated.

Further, the control section 42 performs output control of an ultrasound drive signal so as to make an amplitude of ultrasound vibration in a case of performing treatment by ultrasound vibration to a living tissue constant from an ultrasound drive voltage of an ultrasound drive signal that is measured by the ultrasound voltage measuring section 45, and an ultrasound drive current that is measured by the ultrasound current measuring section 44.

Further, the control section 42 in the present embodiment has a function of a wire breakage detecting section 42b that performs detection of occurrence of wire breakage of the high frequency signal lines 3a and 3b of the treatment instrument 3 (including the cable 7) that is connected to the high frequency output terminal of the power supply apparatus 2 when treatment is performed for a living tissue by a high frequency current and ultrasound vibration. Note that the high frequency signal lines 3a and 3b (including the cable 7) are formed by the high frequency signal lines 7a and 7b inside the cable 7, the high frequency signal lines 12a and 12b inside the main body unit 12 and the high frequency signal lines 9a and 9b of the handle unit 9.

In more detail, the high frequency signal line 3a is formed by the high frequency signal line 7a inside the cable 7, the high frequency signal line 12a inside the main body unit 12, and the high frequency signal line 9a of the handle unit 9, and the high frequency signal line 3b is formed by the high frequency signal line 7b inside the cable 7, the high frequency signal line 12b inside the main body unit 12 and the high frequency signal line 9b of the handle unit 9.

When occurrence of wire breakage of the high frequency signal lines 3a and 3b of the treatment instrument 3 is detected, it is theoretically possible to detect that wire breakage occurs in the high frequency signal lines 3a and 3b from the high frequency current that flows to the treatment instrument 3 side.

However, when treatment is applied to a living tissue by a high frequency current and ultrasound vibration, a portion of the living tissue that is grasped by the grasping members 15a and 15b can be equivalently approximated by resistance. The resistance changes in such a manner that a resistance value thereof becomes larger as moisture is evaporated with treatment.

Resistance changes with treatment as above, and therefore, when occurrence of wire breakage is determined from measurement of a resistance value, the reliability or precision thereof is likely to be reduced.

In contrast with this, as for an electrostatic capacity between the high frequency signal lines 3a and 3b of the treatment instrument 3, a value of the electrostatic capacity is sufficiently small and change thereof is also small in most cases even when moisture is evaporated when a living tissue is grasped with the grasping members 15a and 15b and subjected to treatment. Therefore, the wire breakage detecting section 42b in the present embodiment detects presence or absence of wire breakage in the high frequency signal lines 3a and 3b of the treatment instrument 3, or occurrence of wire breakage with high precision by calculating or measuring the electrostatic capacity value between the high frequency signal lines 3a and 3b of the treatment instrument 3.

The wire breakage detecting section 42b has a calculation section 42c that calculates an electrostatic capacity value Cm between the high frequency signal lines 3 a and 3b of the treatment instrument 3 including the cable 7 from the high frequency output terminal, from the high frequency voltage that is measured by the above described high frequency voltage measuring section 39, and the high frequency current that is measured by the high frequency current measuring section 37.

Further, the wire breakage detecting section 42b has a determination section 42d that determines presence or absence of wire breakage by comparing a threshold value Cth that is set at a value smaller than at least an electrostatic capacity value C which the high frequency signal lines 3a and 3b of the treatment instrument 3 including the cable 7 connected to the high frequency output terminal have in a state without wire breakage, and the electrostatic capacity value Cm that is actually calculated (namely, comparing whether or not Cm < Cth is satisfied).

Further, the power supply apparatus 2 has a threshold value setting section 48 that sets a value of the threshold value Cth that is outputted to the above described determination section 42d. The threshold value setting section 48 has a nonvolatile memory 48a that stores, for example, threshold values Cth1, Cth2, ... and Cthn of a plurality of values in the threshold value setting section 48, and a user such as a surgeon can select and set a threshold value Cthi of a value corresponding to the treatment instrument 3 that is used when performing a surgical operation as the threshold value Cth.

Further, when the control section 42 obtains a determination result of occurrence of wire breakage, namely, that wire breakage is present by the determination section 42d, the control section 42 quickly outputs control signals that stop generation of high frequency power and an ultrasound drive power to the high frequency power generating section 32 and the ultrasound power generating section 33, and the high frequency power generating section 32 and the ultrasound power generating section 33 respectively perform control of stopping generation of high frequency power and ultrasound drive power.

Further, in the case of the determination result that wire breakage is present, the control section 42 outputs a signal that causes the facts that wire breakage occurs, and generation of the high frequency power and the ultrasound drive power is stopped due to occurrence of wire breakage to be displayed to a display section 49. Subsequently, the display section 49 displays occurrence of wire breakage corresponding to the determination result by the determination section 42d, and performs display of stoppage of generation of the high frequency power and the ultrasound drive power. The user such as a surgeon is enabled to grasp the facts that wire breakage occurs and output is stopped due to the wire breakage from the display content of the display section 49, quickly. Note that the display section 49 may also display information of a determination result that wire breakage does not occur by the determination section 42d.

In Fig. 2, the wire breakage detecting section 42b is provided inside the control section 42, but the control section 42 and the wire breakage detecting section 42b may be configured to be in different blocks. Further, the calculation section 42c and the determination section 42d may be configured to be in different blocks from the control section 42.

As shown in Fig. 1, the power supply apparatus 2 is provided with a power supply switch 50, and when the power supply switch 50 is turned on, the power supply section 31 supplies a direct-current power supply for operation to the respective sections inside the power supply apparatus 2.

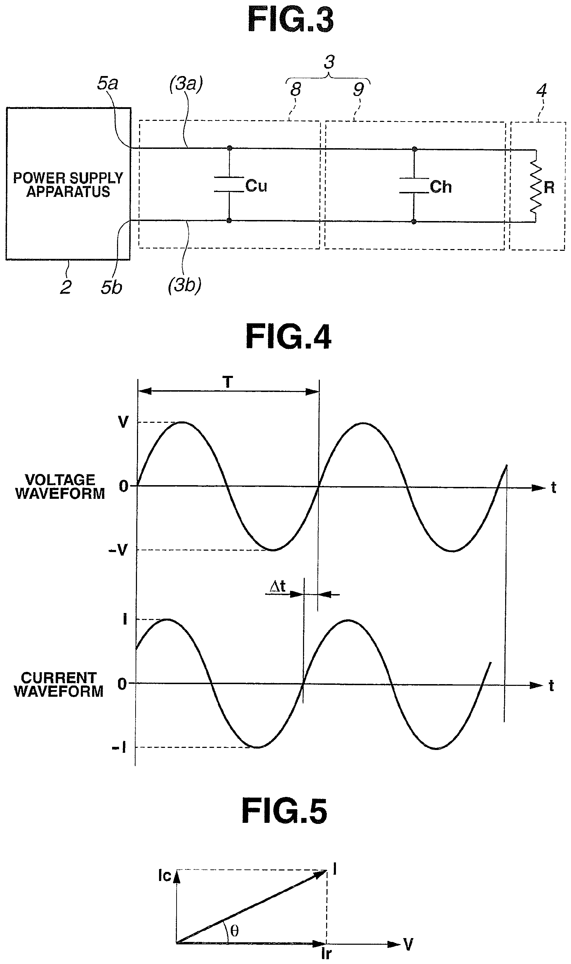

Fig. 3 shows an equivalent circuit of the high frequency signal lines 3a and 3b of the treatment instrument 3 including the cable 7 that is connected to the high frequency output terminal of the power supply apparatus 2.

As shown in Fig. 3, both the high frequency signal lines 3a and 3b inside the treatment instrument 3 including the cable 7 are disposed in positions close to each other via an insulating material inside the cable 7 and the like, and therefore, the electrostatic capacity thereof can be approximated as equivalent to the electrostatic capacity between both the high frequency signal lines 3a and 3b.

Therefore, the high frequency signal lines 3a and 3b can be approximated as equivalent to the result of an electrostatic capacity (value) Cu inside the ultrasound transducer unit 8, and an electrostatic capacity (value) Ch inside the handle unit 9 being connected in parallel.

Further, when a living tissue containing moisture such as the blood vessel 4 or the like is grasped by the grasping members 15a and 15b, the high frequency signal lines 3a and 3b can be approximated as equivalent to a resistance (value) R expressing the resistance component of the grasped living tissue portion being connected in parallel to the electrostatic capacity values Cu and Ch that are connected in parallel.

Therefore, the high frequency signal lines 3a and 3b of the treatment instrument 3 including the cable 7 connected to the high frequency output terminal where the high frequency voltage and the high frequency current are measured can be approximated as equivalent to the electrostatic capacity values Cu and Ch and a resistance R being connected in parallel to the high frequency output terminal.

Note that in Fig. 3, (3a) and (3b) represent signal lines in the equivalent circuit that correspond to the high frequency signal lines 3a and 3b.

As described above, since a living tissue contains moisture, the resistance R has a small value immediately after the living tissue is grasped by the grasping members 15a and 15b, but while treatment such as coagulation and dissection, or sealing is performed, the moisture is evaporated from the living tissue, and therefore, the resistance R becomes large.

When impedance of the high frequency signal lines 3a and 3b is measured at contact points 5a and 5b sides, contribution of the resistance R component is so large that the electrostatic capacity values Cu and Ch can be ignored at the time of start of treatment. As the value of the resistance R becomes larger with progress of the treatment, the impedance becomes that of the electrostatic capacity values Cu and Ch being connected in parallel to the resistance R.

In a state in which the living tissue is not grasped by the grasping members 15a and 15b, or in a state in which the grasping members 15a and 15b are opened, the resistance R can be approximated as infinity. In the state, the impedance of the high frequency signal lines 3a and 3b can be approximated by the result of the electrostatic capacity values Cu and Ch being connected in parallel.

Fig. 4 shows a schematic diagram of output waveforms of the high frequency signal that is outputted from the high frequency output terminal to the treatment instrument 3 side.

In the state in which the electrostatic capacity values Cu and Ch and the resistance R are connected in parallel as shown in Fig. 3, when a high frequency signal is outputted to the high frequency signal lines 3a and 3b of the treatment instrument 3 including the cable 7 from the high frequency output terminal of the power supply apparatus 2, a phase difference $\theta$ having a time difference $\Delta t$ occurs at timing of zero crossing between a high frequency voltage waveform and a high frequency current waveform as shown in Fig. 4.

The calculation section 42c inside the control section 42 shown in Fig. 2 calculates the time difference $\Delta t$ and a period T shown in Fig. 4 from the high frequency voltage that is measured by the high frequency voltage measuring section 39 and is inputted via the A/D converter 41, and the high frequency current that is measured by the high frequency current measuring section 37.

Further, the calculation section 42c calculates the phase difference $\theta$ from the time difference $\Delta t$ and the period T by the following equation (1).

$$\theta = 360° \times \Delta t/T \qquad (1)$$

Fig. 5 shows a vector diagram of the high frequency voltage and the high frequency current that correspond to Fig. 4. As shown in Fig. 5, an amplitude V of the high frequency voltage and an amplitude I of the high frequency current have the phase difference θ.

The calculation section 42c calculates a high frequency current component Ic that flows in the electrostatic capacity values Cu and Ch (namely, Cu + Ch) that are connected in parallel, and a high frequency current component Ir that flows in the resistance R by the following equation (2) and equation (3).

$$Ic = \sin \theta \times I \qquad (2)$$

$$Ir = \cos \theta \times I \qquad (3)$$

Further, the calculation section 42c calculates a synthesized electrostatic capacity value Cm (= Cu + Ch) of the electrostatic capacity values Cu and Ch that are connected in parallel by the following equation (4).

$$C = Ic/\{2\pi \times (1/T) \times V\} \qquad (4)$$

Further, the calculation section 42c calculates the resistance R by the following equation (5).

$$R = V/Ir \qquad (5)$$

The calculation section 42c outputs the synthesized electrostatic capacity value Cm that is calculated to the determination section 42d. The determination section 42d compares the synthesized electrostatic capacity value Cm that is calculated by the calculation section 42c, and the threshold value Cth that is set to detect wire breakage by the threshold value setting section 48, and the determination section 42d determines that wire breakage is absent in a case of Cm ≥ Cth, whereas in a case of Cm < Cth, the determination section 42d determines that wire breakage is present.

When the determination section 42d determines that wire breakage is absent, the control section 42 keeps an usual use state, whereas when the determination section 42d determines that wire breakage is present, the control section 42 performs output control so as not to output a high frequency signal and an ultrasound drive signal from the power supply apparatus 2, displays the content by the display section 49 and notifies the surgeon thereof. The surgeon can quickly recognize occurrence of wire breakage by the notification, and easily perform treatment corresponding to occurrence of wire breakage smoothly.

As above, the surgical operation system 1 of the present embodiment has the power supply apparatus 2 that includes the high frequency power generating section 32 (the first signal producing section) that produces a high frequency signal (the first signal) to supply high frequency energy to a living tissue, and the ultrasound power generating section 33 (the second signal producing section) that produces an ultrasound drive signal (the second signal) to supply ultrasound energy that is energy different from the high frequency energy to the living tissue.

Further, the surgical operation system 1 has the (high frequency/ultrasound) treatment instrument 3 as the surgical treatment instrument that has the ultrasound transducer 11 as the converter that generates the aforesaid other energy by supply of the aforesaid second signal, and the grasping members 15a and 15b that openably and closably grasp a living tissue of an object to be treated, and is capable of simultaneously performing treatment by the aforesaid high frequency energy for the aforesaid living tissue via the pair of electrodes that are formed by the aforesaid grasping members 15a and 15b, and treatment by the aforesaid other energy via the aforesaid grasping members 15a and 15b, and the cable 7 that has one end connected to the aforesaid surgical treatment instrument, and the other end provided with the connector 6 that is detachably connected to the aforesaid power supply apparatus 2, and supplies the aforesaid first signal and the aforesaid second signal from the aforesaid power supply apparatus 2 to the aforesaid surgical treatment instrument via the aforesaid connector 6.

Further, the surgical operation system 1 has the high frequency voltage measuring section 39 that is provided in the aforesaid power supply apparatus 2, and measures the high frequency voltage of the aforesaid high frequency signal that is outputted from the aforesaid power supply apparatus 2 to the side of the second high frequency signal line that electrically continues to the aforesaid pair of electrodes in the aforesaid surgical treatment instrument via the first high frequency signal line in the aforesaid cable 7, the high frequency current measuring section 37 that is provided in the aforesaid power supply apparatus 2, and measures the high frequency current of the aforesaid high frequency signal that is outputted from the aforesaid power supply apparatus 2 to the side of the aforesaid second high frequency signal line in the aforesaid surgical treatment instrument via the aforesaid first high frequency signal line in the aforesaid cable 7, the calculation section 42c that is provided in the aforesaid power supply apparatus 2, and calculates the electrostatic capacity value Cm that the aforesaid first high frequency signal line in the aforesaid cable 7 that transmits the aforesaid high frequency signal and the second high frequency signal line in the aforesaid surgical treatment instrument have, on the basis of the aforesaid high frequency voltage that is measured by the aforesaid high frequency voltage measuring section 39 and the aforesaid high frequency current that is measured by the aforesaid high frequency current measuring section 37, in the middle of treating the aforesaid living tissue by the aforesaid high frequency energy and the aforesaid other energy, the determination section 42d that is provided in the aforesaid power supply apparatus 2, and determines whether or not the aforesaid electrostatic capacity value Cm that is calculated by the aforesaid calculation section 42c is smaller than the threshold value Cth that is set in advance to detect occurrence of wire breakage in the aforesaid first high frequency signal line in the aforesaid cable 7 and the aforesaid second high frequency signal line in the aforesaid surgical treatment instrument, and the control section 42 that is provided in the aforesaid power supply apparatus 2, and performs control of stopping output of the aforesaid high frequency signal and the aforesaid second signal that are supplied from the aforesaid power supply apparatus 2 to the aforesaid surgical treatment instrument via the aforesaid cable 7 when the aforesaid determination section 42d determines that the aforesaid electrostatic capacity value Cm is smaller than the aforesaid threshold value Cth.

Next, an operation according to the surgical operation system 1 of the present embodiment will be described.

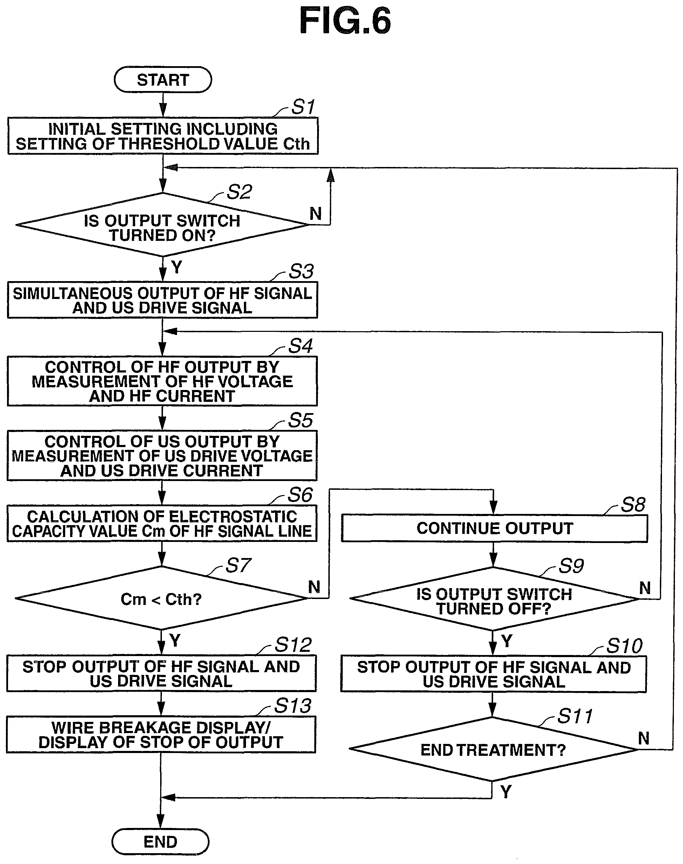

Fig. 6 shows a flowchart of a processing procedure mainly by the control section 42 in a case of performing treatment according to the surgical operation system 1 of the present embodiment.

The power supply switch 50 of the power supply apparatus 2 is turned on, whereby the respective sections in the power supply apparatus 2 are brought into an operation state. A surgeon performs initial setting of a high frequency output value, an ultrasound drive output value and the like including setting of the threshold value Cth in the first step S1.

Fig. 7A shows examples of the electrostatic capacity value Ca in a normal case in which wire breakage in the treatment instrument 3 including the cable 7 does not occur, and the electrostatic values Cb and Cc in a case in which wire breakage occurs.

A horizontal axis in Fig. 7A shows coarse electrostatic capacity values corresponding to lengths of the high frequency signal lines 7a and 7b in the cable 7, the high frequency signal lines 12a and 12b in the main body unit 12, and the high frequency signal lines 9a and 9b in the handle unit 9 that configure the high frequency signal lines 3a and 3b. Note that the electrostatic capacity values Ca, Cb and Cc shown in Fig. 7A are measured (calculated) in end portions at the connector 6 side in the high frequency signal lines 7a and 7b of the cable 7.

The high frequency signal lines 7a and 7b inside the cable 7 usually have lengths of approximately 3 m, and the high frequency signal lines 9a and 9b inside the handle unit 9 have lengths of about several tens centimeters. Further, the high frequency signal lines 12a and 12b inside the main body unit 12 are considerably shorter than the lengths in the case of the handle unit 9.

In the case without wire breakage, the electrostatic capacity value Ca in the normal case is calculated by the calculation section 42c on the basis of the measurement result of the high frequency voltage and the high frequency current. The electrostatic capacity value Ca is usually in a range of about 250 pF to 300 pF. Therefore, the value of the threshold value Cth is also set at a value substantially within a range of about 250 pF to 300 pF. In response to a case in which the cable 7 is longer, the value of the threshold value Cth may be set at a value within a range of about 250 pF to 400 pF, and in response to a case in which the cable 7 is shorter, the value of the threshold value Cth may be set at a value within a range of about 150 pF to 400 pF.

When wire breakage occurs to positions shown by cross marks B in Fig. 7A, the electrostatic capacity values are like the electrostatic capacity values Cb and Cc (smaller than the electrostatic capacity value Ca in the case without wire breakage) that correspond to the wire breakage positions from the end portion (namely, the high frequency output terminal) at the connector 6 side of the cable 7.

Therefore, if the threshold value Cth for detecting wire breakage is set at a value Cth1 that is slightly smaller than the electrostatic capacity value Ca in the case of without wire breakage, occurrence of wire breakage at an optional position in the treatment instrument 3 (including the cable 7) can be detected by comparison with the electrostatic capacity value Cm that is calculated. Note that in Fig. 7A, Cth1 is set at a value smaller than the electrostatic capacity value Ca by a margin $\Delta$.

A surgeon can set the threshold value Cth by the threshold value setting section 48 in response to the electrostatic capacity value in the case of the treatment instrument 3 that is used in the case of actually performing treatment. The set threshold value Cth is used when presence or absence of wire breakage is determined by the determination section 42d.

When the surgeon seals, for example, the blood vessel 4 as a living tissue of an object to be treated, the surgeon grasps the blood vessel 4 with the grasping members 15a and 15b, and turns on the output switch 24.

As shown in step S2 of Fig. 6, the control section 42 monitors ON of the output switch 24, and when the output switch 24 is turned on, the control section 42 controls the high frequency power generating section 32 and the ultrasound power generating section 33 so as to output a high frequency signal and an ultrasound drive signal simultaneously as shown in step S3. Note that in Fig. 6, the high frequency signal is abbreviated as an HF signal, and the ultrasound drive signal is abbreviated as a US drive signal.

In this case, the high frequency signal flows to the blood vessel 4 that is grasped by the grasping members 15a and 15b that configure a bipolar electrode via the high frequency signal lines 3a and 3b in the treatment instrument 3 including the cable 7 from the high frequency output terminal, and treatment of sealing by high frequency energy for the blood vessel 4 is started.

Further, the ultrasound transducer 11 performs ultrasound vibration by the ultrasound drive signal, and the ultrasound vibration is given to the blood vessel 4 via the grasping member 15a, and treatment of sealing is also started for the blood vessel 4 by the ultrasound vibration (ultrasound energy). Both kinds of energy are simultaneously used in this manner, whereby the function of sealing is increased, and treatment of sealing can be performed in a short time.

Further, as shown in step S4, the high frequency voltage measuring section 39 and the high frequency current measuring section 37 respectively measure the high frequency voltage and the high frequency current that are supplied from the high frequency output terminal to the treatment instrument 3 side. The high frequency voltage measuring section 39 and the high frequency current measuring section 37 output the high frequency voltage and the high frequency current that are measured to the control section 42 via the A/D converter 41. The control section 42 calculates the resistance component of the living tissue from the high frequency voltage and the high frequency current that are measured, and performs output control of the high frequency signal to provide a high frequency energy value suitable for the case of treating the living tissue.

Further, as shown in step S5, the ultrasound voltage measuring section 45 and the ultrasound current measuring section 44 respectively measure the ultrasound drive voltage and the ultrasound drive current, and output the ultrasound drive voltage and the ultrasound drive current to the control section 42 via the A/D converter 41. The control section 42 controls ultrasound drive power of the ultrasound power generating section 33 so that the amplitude of the ultrasound vibration generated by the ultrasound transducer 11 becomes constant, from the measured values.

Further, as shown in step S6, the calculation section 42c calculates the electrostatic capacity value Cm between the high frequency signal lines 3a and 3b from the high frequency voltage and the high frequency current that are inputted, and outputs the electrostatic capacity value Cm to the determination section 42d. Note that the processing of step S6 may be performed in parallel with the processing of step S5, or before the processing of step S5.

As shown in step S7, the determination section 42d compares the calculated electrostatic capacity value Cm and the threshold value Cth, and determines whether or not the electrostatic capacity value Cm satisfies the condition of Cm < Cth. When the condition is not satisfied, the determination section 42d determines that wire breakage is absent. In the case of the determination result by the determination section 42d, the control section 42 performs control so as to continue a state of simultaneously outputting the high frequency signal and the ultrasound drive signal as shown in step S8.

Further, as shown in step S9, the control section 42 monitors whether or not the output switch 24 is turned off. When the output switch 24 is not turned off, the control section 42 returns to the processing of step S4.
When the output switch 24 is turned off, the control section 42 performs control to stop simultaneous output of the high frequency signal and the ultrasound drive signal as shown in step S10, and proceeds to the next step S 11.
In step S11, the control section 42 monitors whether or not an instruction operation of ending the treatment is performed, and when the instruction operation of ending the treatment is not performed, the control section 42 returns to the processing of step S2. When the instruction operation of ending the treatment is performed, the control section 42 ends the control operation of Fig. 6, and turns off the power supply switch 50.

When the condition of Cm < Cth is satisfied in step S7, the determination section 42d determines that wire breakage is present, and in the case of the determination result, the control section 42 controls the high frequency power generating section 32 and the ultrasound power generating section 33 to stop simultaneous output of the high frequency signal and the ultrasound drive signal as shown in step S12.

Further, as shown in step S13, the control section 42 outputs a signal to cause the facts that wire breakage occurs and output of the high frequency signal and the ultrasound drive signal is stopped to be displayed to the display section 49. Subsequently, the display section 49 displays occurrence of wire breakage, and stop of output of the high frequency signal and the ultrasound drive signal, and the control section 42 disables simultaneous output of the high frequency signal and the ultrasound drive signal until the portion where wire breakage occurs is replaced. Note that the processing of step S12 and the processing of step S13 may be simultaneously performed, or the processing of step S12 may be performed after the processing of step S13 is performed.

The surgeon can quickly grasp that wire breakage occurs, and also can grasp that due to occurrence of wire breakage, the power supply apparatus 2 is brought into a cutoff state. The surgeon is able to easily perform the processing corresponding to the occurrence of wire breakage smoothly.

As above, according to the present embodiment, the high frequency voltage and the high frequency current that are supplied to the treatment instrument 3 are measured, the electrostatic capacity value Cm of the treatment instrument 3 is calculated on the basis of the high frequency voltage and the high frequency current that are measured, and the calculated electrostatic capacity value Cm is compared with the threshold value Cth, whereby when wire breakage occurs to the high frequency signal lines 3a and 3b of the treatment instrument 3, occurrence of wire breakage can be quickly detected.

Further, the electrostatic capacity value Cm of the treatment instrument 3 is calculated on the basis of the high frequency voltage and the high frequency current that are measured, and the calculated electrostatic capacity value Cm is compared with the threshold value Cth for determining wire breakage, whereby occurrence of wire breakage is determined (detected), and therefore, occurrence of wire breakage can be determined with high precision.

Further, according to the present embodiment, when occurrence of wire breakage is detected, output of the high frequency signal and the ultrasound drive signal is stopped, and the surgeon is notified of the occurrence of wire breakage. Therefore, an effect of enabling the surgeon to perform treatment corresponding to the occurrence of wire breakage quickly is provided.

As described above, when a surgical operation is performed with use of both the high frequency energy and the ultrasound energy, the state in which treatment by the ultrasound energy can be performed continues even if wire breakage occurs, and therefore, it becomes difficult for the surgeon to recognize the occurrence of wire breakage immediately. Further, the state differs from the output state that is set so that proper treatment is performed in the case of use of both the high frequency energy and the ultrasound energy, and therefore, it is desirable to stop the treatment quickly.

In the present embodiment, when wire breakage occurs, a surgeon is immediately notified of the occurrence of wire breakage, and output of high frequency energy and ultrasound energy is prevented. Therefore, the surgeon can smoothly proceed with processing or the like corresponding to occurrence of wire breakage such as continuing treatment by replacing the treatment instrument 3 where wire breakage occurs, with respect to the occurrence of wire breakage.

Note that as described above, when the treatment instrument 3 is used for one case, the possibility of occurrence of wire breakage in the handle unit 9 as which new one is used at each case is extremely low, as compared with the case of the ultrasound transducer unit 8 that is repeatedly used.

Therefore, the threshold value Cth for detecting occurrence of wire breakage may be set so that only the case in which wire breakage occurs to the ultrasound transducer unit 8 side can be detected in some cases.

Thus, as shown in Fig. 7A, the value of Cth2 may be set as the threshold value Cth. The value Cth2 can be set at a value slightly smaller than the electrostatic capacity value Cu of the high frequency signal lines 8a and 8b of the ultrasound transducer unit 8. In this case, with the degree of variations in the products of the ultrasound transducer units 8 taken into consideration, a value of Cu-ΔCu that is the result of subtracting a maximum capacity value ΔCu due to the variations from the electrostatic capacity value Cu as the threshold value Cth.

Further, as a modification of the present embodiment, the following configuration may be adopted.

For example, as shown by the dotted lines in Fig. 2, a timer 51 as a time measuring section that performs time measurement, and a storage section 52 that stores in time series the electrostatic capacity value Cm(j) that is calculated by (the calculation section 42c of) the control section 42 on the basis of a high frequency voltage and a high frequency current that are measured at a measurement time tj (j is a natural number, j=1, 2, ...) at each predetermined time δ, for example, may be provided in the power supply apparatus 2. Further, the determination section 42d compares an absolute value |Cm(j) - Cm(j+1)| of a difference value of two electrostatic capacity values Cm(j) and Cm(j+1) that are measured (calculated) at the predetermined time δ, and by being shifted in terms of time with a threshold value Cth(δ) that is set as follows. Note that the threshold value Cth(δ) is a positive value.

The absolute value |Cm(j) - Cm(j+1)| becomes a small value due to a measurement error and a change of state of a living tissue in a normal operation state of the treatment instrument 3.

With respect to the case as above, the threshold value Cth(δ) that satisfies

$$|\mathrm{Cm(j)} - \mathrm{Cm(j+1)}| < \mathrm{Cth}(\delta) \qquad (6)$$

can be set. As the threshold value Cth(δ), a maximum value of a change amount of the electrostatic capacity value that is allowed when the electrostatic capacity value Cm is measured at time intervals of the predetermined times δ can be set in the normal operation state.

The threshold value Cth(δ) becomes a sufficiently small value (in the following example, 1/10 or less) as compared with the value of the threshold value Cth described above, though it depends on the length of the predetermined time δ.

For example, when the predetermined time δ is set at a value within a range of 0.1 seconds to 10 seconds, the threshold value Cth(δ) can be set from a range of Cm/100 - Cm/10 in accordance with a use environment. Cm/100 - Cm/10 is within a range of about 2pF to 30pF. The threshold value Cth(δ) can be set by, for example, the threshold value setting section 48. In this case, the threshold value setting section 48 has a function of a second threshold value setting section that sets the threshold value Cth(δ) as a second threshold value when the threshold value setting section 48 is assumed to be a first threshold value setting section that sets the threshold value Cth as a first threshold value. Note that when the threshold value setting section 48 already includes the functions of setting the first threshold value and the second threshold value, the threshold value setting section 48 has a function of a third threshold value setting section that sets the threshold value Cth(δ) as a third threshold value. Note that the third threshold value setting section may be provided separately from the threshold value setting section 48.

In the case in which the threshold value Cth(δ) is set as above, the absolute value |Cm(j) - Cm(j+1)| significantly changes to be the threshold value Cth(δ) or more if wire breakage occurs. Therefore, the determination section 42d determines whether or not the absolute value |Cm(j) - Cm(j+1)|is smaller than the threshold value Cth($\delta$), namely, whether or not the condition of expression (6) is satisfied, and thereby can determine (detect) occurrence of wire breakage. Note that the determination section 42d also performs the above described determination, but, for example, a second determination section different from the determination section 42d may perform the above described determination.

Further, when an abnormal operation state other than wire breakage occurs, the absolute value |Cm(j) - Cm(j+1)| is likely to increase to be the above described threshold value Cth($\delta$) or larger (namely, be brought into a state in which expression (6) is not satisfied).

In the present modification, except that the determination section 42d performs determination as in the above described first embodiment, the determination section 42d determines whether or not expression (6) is satisfied. When expression (6) is not satisfied, the control section 42 also performs control of stopping output of the high frequency signal and the ultrasound drive signal and notifies a surgeon of it as at the time of detecting occurrence of wire breakage described above. As notification in this case, the control section 42 notifies the surgeon that an abnormal operation state is brought about.

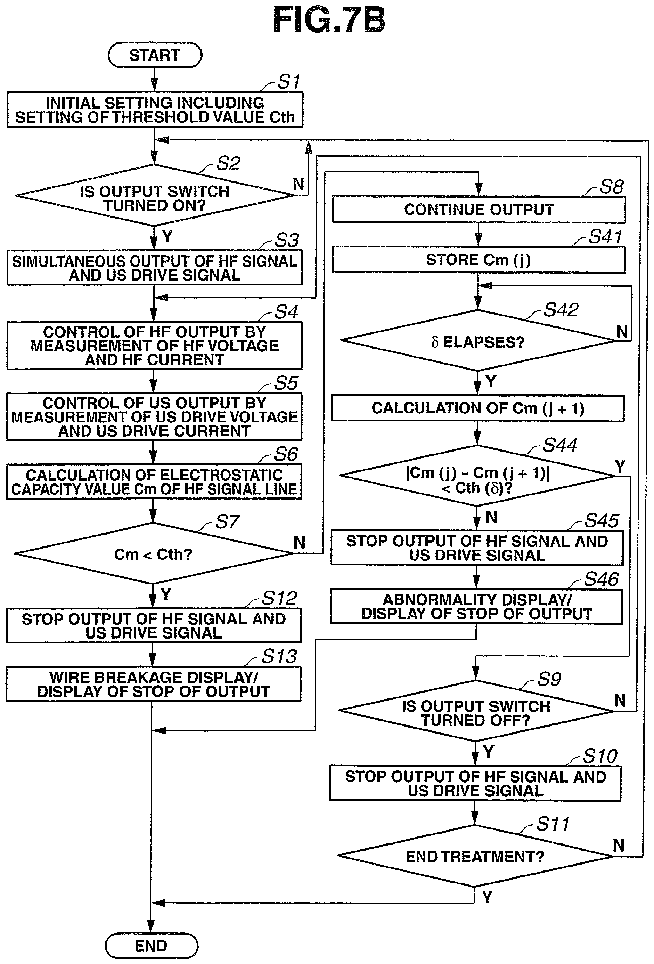

Fig. 7B shows a flowchart that is a typical example of a processing content of the present modification. Fig. 7B differs in a part in the flowchart shown in Fig. 6, and therefore only the different part will be described. Steps S1 to S5 are similar to the case of Fig. 6, the processing of calculation of the electrostatic capacity value Cm between the high frequency signal lines 3a and 3b in step S6 in Fig. 6 is changed to that of the electrostatic capacity value Cm(j) that is calculated at the measurement time Tj (j=1 in this case) as shown in Fig. 7B. Further, the processing of determining whether or not the condition of Cm < Cth is satisfied in step S7 of Fig. 6 becomes the processing of determining whether or not the condition of Cm(j) < Cth is satisfied in Fig. 7B. Further, in Fig. 7B, the following processing is performed between steps S8 and S9.

In step S41 after the processing of step S8, the control section 42 stores the electrostatic capacity value Cm(j) with the measurement time Tj in the storage section 52. In the next step S42, the timer 51 waits until the predetermined time $\delta$ elapses from the measurement time tj. When the predetermined time $\delta$ elapses, the timer 51 notifies (the calculation section 42c of) the control section 42 of the lapse of the predetermined time $\delta$. The timer 51 notifies (the calculation section 42c of) the control section 42 that the next measurement time tj+1 arrives. In step S43, (the calculation section 42c of) the control section 42 recognizes that the next measurement time tj+1 when the predetermined time $\delta$ elapses from the measurement time tj arrives (sets j at j+1), calculates the electrostatic capacity value Cm(j+1) at the measurement time tj+1, and sends the calculated electrostatic capacity value Cm(j+1) to the determination section 42d.

In step S44, (the determination section 42d of) the control section 42 performs determination of whether the condition of expression (6) is satisfied. Namely, (the determination section 42d of) the control section 42 performs determination of whether or not |Cm(j) - Cm(j+1)| < Cth($\delta$) is satisfied. When expression (6) is satisfied, the control section 42 proceeds to the processing of step S9. When the output switch 24 is not turned off in step 9, the control section 42 returns to the processing of step S4, and in a state in which j becomes j+1, the aforementioned processing is repeated.

In a case of the determination result that the condition of expression (6) is not satisfied in step S44, the control section 42 controls the high frequency power generating section 32 and the ultrasound power generating section 33 so as to stop simultaneous output of the high frequency signal and the ultrasound drive signal in step S45.

Further, as shown in step S46, the control section 42 outputs a signal that causes the facts that abnormality occurs and output of the high frequency signal and the ultrasound drive signal is stopped to be displayed to the display section 49. Subsequently, the display section 49 displays occurrence of abnormality such as wire breakage, and stop of output of the high frequency signal and the ultrasound drive signal, and the control section 42 disables output until the treatment instrument where wire breakage occurs is replaced, and ends the processing of Fig. 7B. Note that the processing of step S45 and the processing of step S46 may be simultaneously performed, or the processing of step S45 may be performed after the processing of step S46 is performed. Note that in Fig. 7B, step S41 may be performed simultaneously with step S8, or may be performed before step S8.

According to the present modification, occurrence of wire breakage can be more reliably detected, and when an abnormal state other than wire breakage is brought about, stop of output of the high frequency signal and the ultrasound drive signal can be performed. Further, the surgeon can be quickly informed of the occurrence of an abnormal state.

(Second Embodiment)

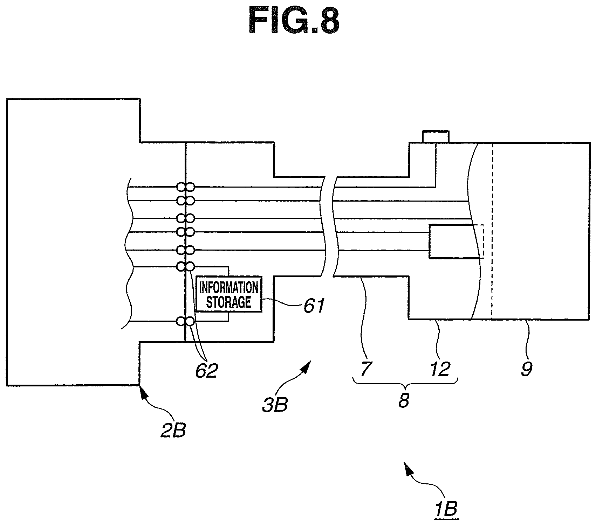

Next, a second embodiment of the present invention will be described. Fig. 8 shows a surgical operation system 1B of the second embodiment. The surgical operation system 1B has a power supply apparatus 2B, and a treatment instrument 3B including the connector 6 that is detachably connected to the power supply apparatus 2B.

The treatment instrument 3B has an information storage section 61 that stores information of the electrostatic capacity value Cu which the high frequency signal lines 8a and 8ab of the ultrasound transducer unit 8 including the cable 7 have and which is measured at the time of factory shipment, for example, in the connector 6, in the treatment instrument 3 of the first embodiment.

Note that the electrostatic capacity value Cu is measured (calculated) by an electrostatic capacity measuring apparatus or the like that can measure an electrostatic capacity value with high precision, for example.

The information storage section 61 is connected to a contact point 62 that is provided at the connector 6 so that stored information can be read.

Note that the information storage section 61 is configured by a ROM, an RF-ID, a barcode and other devices.

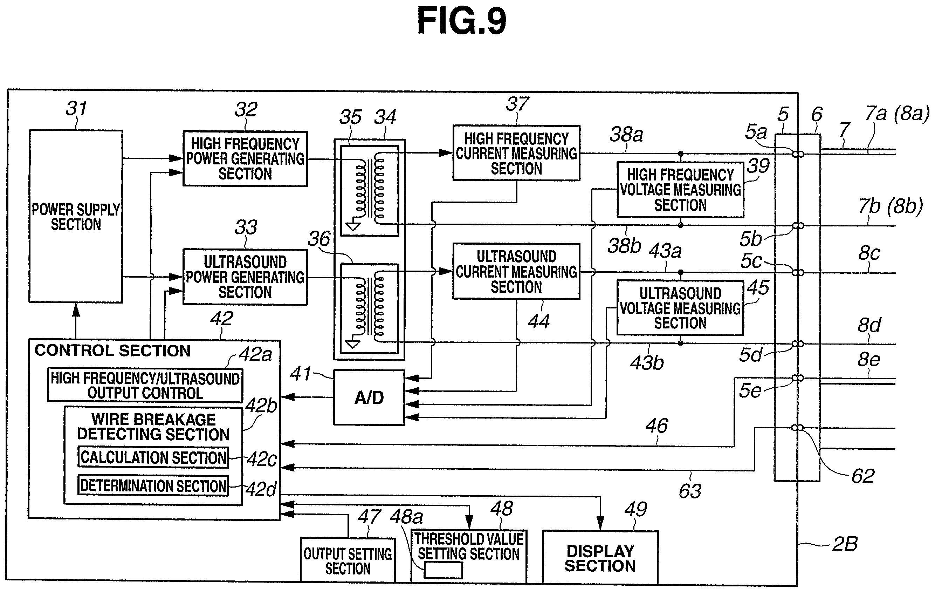

Fig. 9 shows an internal configuration of the power supply apparatus 2B in the present embodiment. In the power supply apparatus 2B, the control section 42 reads the information of the electrostatic capacity value Cu via a signal line 63 that is further connected to the contact point 62 in the power supply apparatus 2 of the first embodiment. Further, the control section 42 sends the information of the electrostatic capacity value Cu to the threshold value setting section 48, so that a threshold value corresponding to the electrostatic capacity value Cu can be set.

In this case, by the threshold value setting section 48, for example, the margin $\Delta C$ is added to the electrostatic capacity value Cu, and as a threshold value corresponding to the electrostatic capacity value Cu, Cu + $\Delta C$ can be set as the threshold value Cth.

The other configuration is similar to that of the first embodiment.

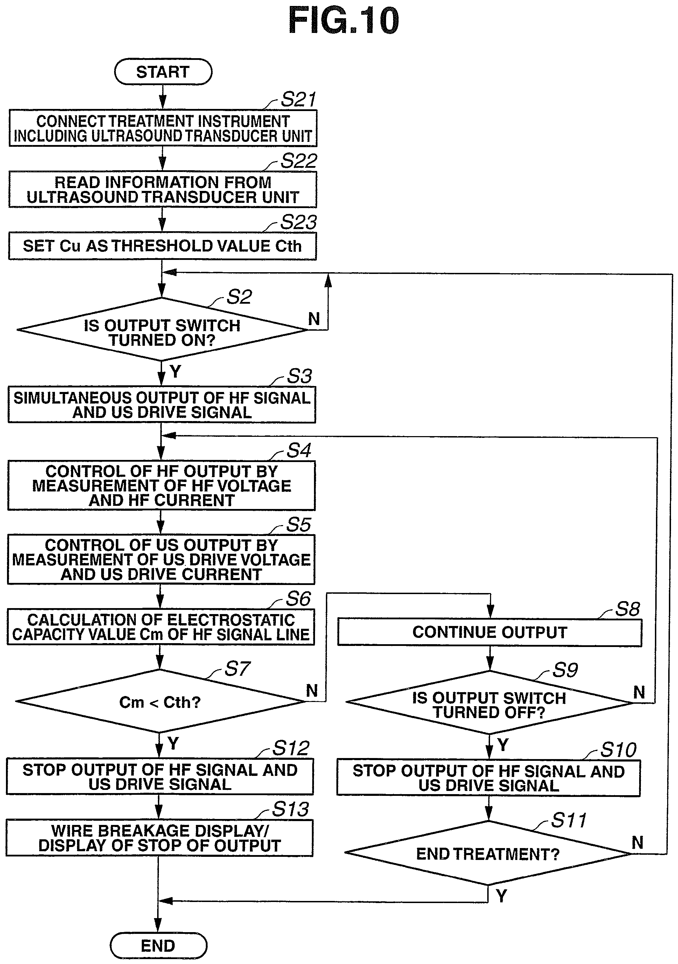

Fig. 10 shows one example of a processing procedure of detecting occurrence of wire breakage in the case in which a surgical operation is performed according to the present embodiment. A processing content shown in Fig. 10 is analogous to the processing content of Fig. 6.

At first, as shown in step S21, the treatment instrument 3B including the ultrasound transducer unit 8 is connected to the power supply apparatus 2B. Thereafter, the power supply switch 50 is turned on. By the power supply switch 50 being turned on, the respective sections inside the power supply apparatus 2 are brought into an operating state. Subsequently, as shown in step S22, the control section 42 reads the electrostatic capacity value Cu as information stored in the information storage section 61 that is provided at the ultrasound transducer unit 8, and sends the electrostatic capacity value Cu to the threshold value setting section 48. In the next step 23, the threshold value setting section 48 sets the electrostatic capacity value Cu as the threshold value Cth, and proceeds to the next step S2. The processing of step S2 and the following steps is similar processing to that in Fig. 6. Therefore, the explanation thereof will be omitted.

Since according to the present embodiment, the electrostatic capacity value Cu of the ultrasound transducer unit 8 that configures the treatment instrument 3B that is actually used when a surgical operation is performed is read, and the electrostatic capacity value Cu is set as the threshold value Cth based on which presence or absence of wire breakage is determined, presence or absence of wire breakage can be determined with high precision. In addition thereto, a similar effect to that of the first embodiment is provided.

(Third Embodiment)

Next, a third embodiment of the present invention will be described. The present embodiment is the surgical operation system 1 that can detect wire breakage with high precision in the case of the treatment instrument 3 of the first embodiment, for example. A hardware configuration is similar to, for example, that of the first embodiment, and the procedure of detecting occurrence of wire breakage differs from the first embodiment.

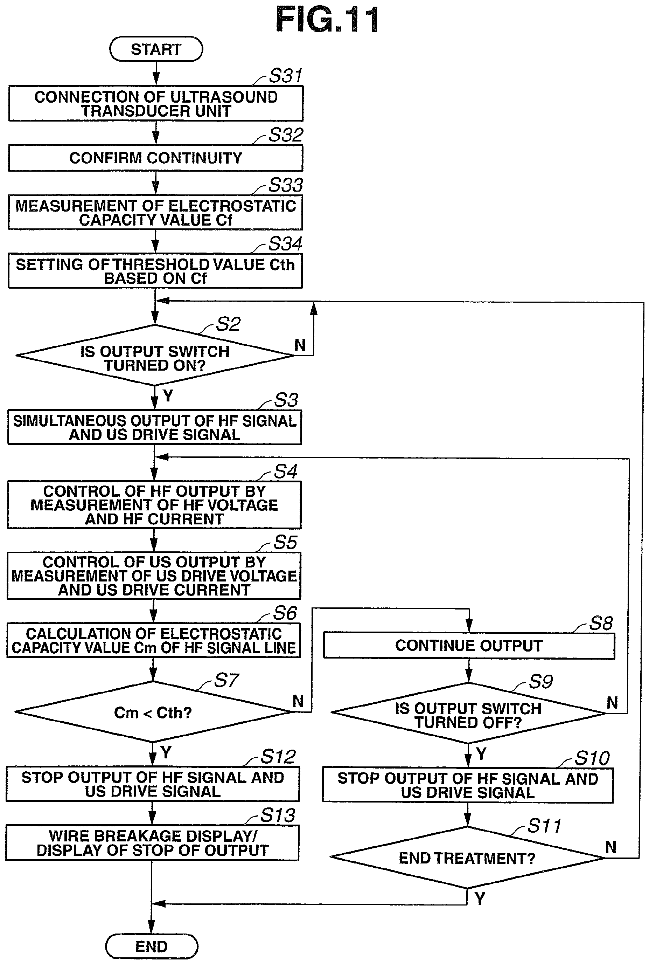

Fig. 11 shows an operation including processing of determining presence or absence of wire breakage in the present embodiment.

In the first step S31, the surgeon connects the ultrasound transducer unit 8 that is actually used in a surgical operation to the power supply apparatus 2, and turns on the power supply switch 50 of the power supply apparatus 2. In the next step S32, the surgeon performs processing of confirming that the ultrasound transducer unit 8 does not have wire breakage, namely, electrical continuity.

For example, when electrical continuity is confirmed for the ultrasound transducer unit 8 as a single piece, both ends of distal ends of the high frequency signal lines 8a and 8b of the ultrasound transducer unit 8 are shorted (short-circuited) by a jig not illustrated.

When electrical continuity is confirmed when the handle unit 9 is fitted to the ultrasound transducer unit 8, the grasping members 15a and 15b at the distal end of the handle unit 9 are shorted with a jig, or the grasping members 15a and 15b are brought into a state in which the grasping members 15a and 15b grasp gauze or the like that is wetted with a physiological saline solution, high frequency signal is outputted, and smoke is produced from the gauze or the like, whereby electrical continuity is confirmed.

In the next step S33, measurement of the electrostatic capacity value Cf of the treatment instrument 3 or the ultrasound transducer unit 8 is performed (to set a threshold value for determining presence or absence of wire breakage during use).

When measurement of the electrostatic capacity value Cf is performed for the ultrasound transducer unit 8 as a single piece, the electrostatic capacity value Cf is set as the threshold value Cth in step S34.

Note that when measurement of the electrostatic capacity value Cf is performed in the treatment instrument 3 in which the handle unit 9 is fitted to the ultrasound transducer unit 8, a value obtained by subtracting an electrostatic capacity value of the handle unit 9 from the electrostatic capacity value Cf is set as the threshold value Cth.

The next processing to step S34 is the same as step S2 of Fig. 6, and since the processing of step S2 and the processing of the following steps are similar to those in Fig. 6, the explanation thereof will be omitted.

Since in the present embodiment, the electrostatic capacity value of the ultrasound transducer unit 8 that is actually used in a surgical operation is measured, and with use of the measured value, the measured value is set as the threshold value Cth based on which presence or absence of wire breakage is determined, presence or absence of wire breakage can be determined with high precision similarly to the case of the second embodiment.

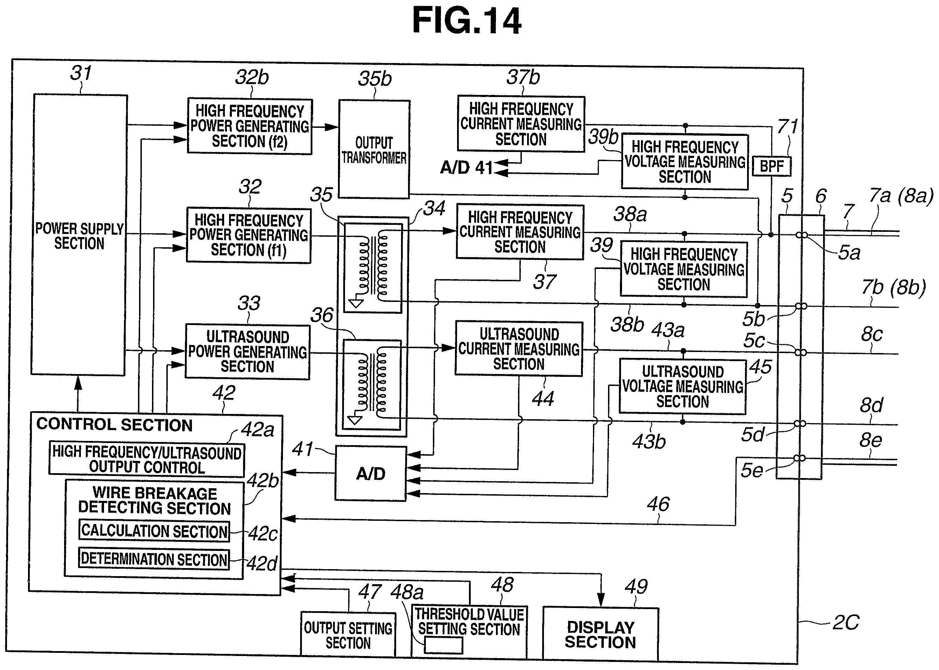

Note that when a configuration that includes a high frequency power generating section 32b or the like that generates a high frequency signal at a frequency f2 that differs from the frequency f1 of the high frequency signal that is generated in the high frequency power generating section 32 is adopted as in a power supply apparatus 2C of Fig. 14 that will be described later, in the first to the third embodiments described above, occurrence of wire breakage may be determined (detected) with the high frequency signals of both the frequencies f1 and f2.

In this case, in a state in which the high frequency signals of both the frequencies f1 and f2 are applied to the treatment instrument 3 (or 3B), electrostatic capacity values Cm(f1) and Cm(f2) are calculated from measured values of high frequency voltages and high frequency currents of the respective high frequency signals of both the frequencies f1 and f2, and the electrostatic capacity values Cm(f1) and Cm(f2) are respectively compared with threshold values Cth(f1) and Cth(f2) for determining occurrence of wire breakage, whereby presence or absence of occurrence of wire breakage may be determined.

Further, when the determination section 42d determines that occurrence of wire breakage is present in the case of use of the frequency f1 that is a higher frequency, similar determination may be performed with use of the frequency f2 that is a lower frequency, and a determination result in the frequency f2 that is a lower frequency may be given a higher priority.

(Fourth Embodiment)

In the first to the third embodiments described above, the explanation is performed, that the electrostatic capacity value is calculated from the measured values of the high frequency voltage and the high frequency current, and presence or absence of occurrence of wire breakage is detected. However, as described as follows (as a fourth embodiment), occurrence of wire breakage may be detected (determined) by impedance Zm being calculated from the measured values of the high frequency voltage and high frequency current of the high frequency signal lines 3a and 3b of the treatment instrument 3 (may be applied to the treatment instrument 3B), and being compared with a threshold value Zth of impedance that is set to detect wire breakage.

Note that a configuration of the fourth embodiment is a configuration similar to that of the first embodiment, for example. However, in the present embodiment, the calculation section 42c of Fig. 2 calculates the impedance Zm, and the determination section 42d of Fig. 2 detects (determines) occurrence of wire breakage by comparing the impedance Zm and the threshold value Zth.

Note that the impedance Zm is calculated from the measured values of the high frequency voltage and the high frequency current, and therefore the impedance Zm is also called measured impedance or measured impedance.

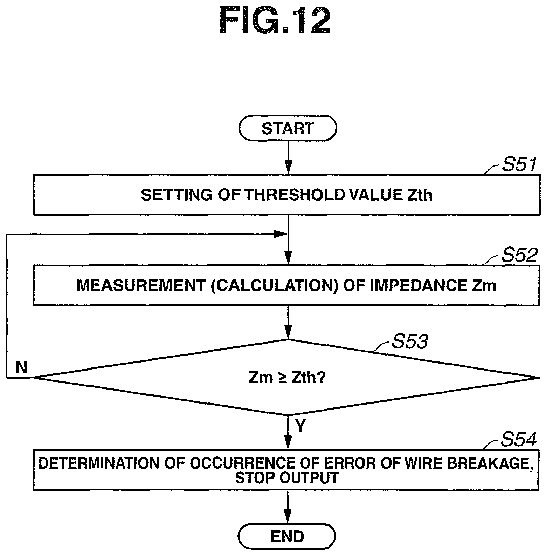

Fig. 12 shows a flowchart of detecting presence or absence of occurrence of wire breakage in this case.

In step S51, the threshold value Zth of impedance for detecting occurrence of wire breakage is set. Note that the handle unit 9 is disposable, and is substantially guaranteed to have no wire breakage in use of one time. Therefore, it is sufficient to detect occurrence of wire breakage at the ultrasound transducer unit 8 side.

When an angular frequency of the ultrasound drive signal is set as $\omega$, impedance in a case of only the ultrasound transducer unit 8 being connected is obtained by $1/(\omega Cu)$.

When wire breakage occurs at the ultrasound transducer unit 8 side, the impedance seen from the high frequency output terminal side is larger than $1/(\omega Cu)$.

As the threshold value Zth, the threshold value Zth is set with use of a value close to a maximum value of the electrostatic capacity value Cu, with consideration given to a variation of the electrostatic capacity value Cu of the ultrasound transducer unit 8 in the impedance $1/(\omega Cu)$ of the ultrasound transducer unit 8.

In the next step S52, the treatment instrument 3 is connected to the power supply apparatus 2, and the control section 42 measures (calculates) the impedance Zm while updating the previous measured value.

In the next step S53, the control section 42 determines whether or not the impedance Zm satisfies a condition of Zm ≥ Zth.

When the impedance Zm does not satisfy the condition of Zm ≥ Zth, the control section 42 returns to the processing of step S52, whereas when the impedance Zm satisfies the condition of Zm ≥ Zth, the control section 42 proceeds to processing of step S54. In step S54, the control section 42 determines that wire breakage is present (error of wire breakage occurs), and performs control to stop output of the high frequency signal and the ultrasound drive signal.

By the processing as shown in Fig. 12 being performed, the impedance Zm that is measured becomes larger than the threshold value Zth and erroneous detection does not occur when wire breakage does not take place even when the distal end of the treatment instrument 3 is set to be in an open state, and occurrence of wire breakage of the ultrasound transducer unit 8 including the cable 7 can be detected with high precision.

While in the case of Fig. 12, the threshold value Zth is set in advance, the threshold value Zth may be set in accordance with the measured value of the ultrasound transducer unit 8 of the treatment instrument 3 that is actually used.

For example, before a surgical operation is performed, the distal end of the treatment instrument 3 is set to be in an open state, the impedance between the high frequency signal lines 3a and 3b of the treatment instrument 3 is measured, and the threshold value Zth is set on the basis of the measured impedance.

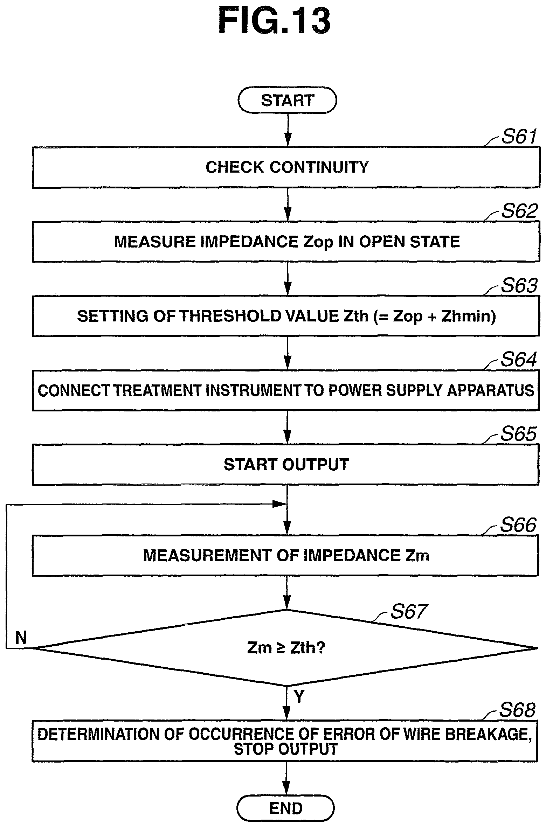

Fig. 13 shows a flowchart of detecting presence or absence of occurrence of wire breakage in the above case.

In the first step S61, only the ultrasound transducer unit 8 is connected to the power supply apparatus 2, the distal end of the ultrasound transducer unit 8 is set to be in a short-circuited state, and the control section 42 performs electrical continuity check of the ultrasound transducer unit 8.

In the next step S62, the ultrasound transducer unit 8 is set to be in an open state that is a state in which the ultrasound transducer unit 8 is not short-circuited, and the control section 42 measures (calculates) impedance Zop in the open state. The impedance Zop in the open state is $1/(\omega Cu)$. Note that when wire breakage occurs to the ultrasound transducer unit 8, the impedance becomes larger than the impedance Zop.

In the next step S63, the control section 42 sets a value obtained by impedance Zhmin set in advance being added to Zop as the threshold value Zth for detecting wire breakage. Namely, Zth = Zop + Zhmin. Note that the impedance Zhmin can be obtained by a difference between the impedance in the case of only the ultrasound transducer unit 8, and the impedance in the case in which the handle unit 9 of the minimum capacity being connected.

Subsequently, in the next step S64, the treatment instrument 3 is connected to the power supply apparatus 2. Namely, the treatment instrument 3 in which the handle unit 9 is fitted to the ultrasound transducer unit 8 is connected to the power supply apparatus 2.

When a surgeon turns on the output switch 24 in the next step S65, the power supply apparatus 2 starts output of the high frequency signal and the ultrasound drive signal.

In the next step S66, the control section 42 performs measurement (calculation) of the impedance Zm from the measured values of the high frequency voltage and the high frequency current (while updating the previous measured values).

In the next step S67, the control section 42 determines whether or not the measured impedance Zm satisfies $Zm \geq Zth$. When the measured impedance Zm does not satisfy the condition of $Zm \geq Zth$, the control section 42 returns to the processing of step S66. When the measured impedance Zm satisfies the condition of $Zm \geq Zth$, the control section 42 proceeds to processing of step S68. In step S68, the control section 42 determines that an error of wire breakage occurs, and performs control to stop output of the high frequency signal and the ultrasound drive signal.

The processing as shown in Fig. 13 is performed, whereby occurrence of wire breakage can be detected with high precision without error detection being performed as a result that the impedance Zm that is measured when wire breakage does not occur even when the distal end is set to be in an open state becomes less than the threshold value Zth.

Fig. 14 shows a configuration of a power supply apparatus 2C that detects occurrence of wire breakage by using two frequencies in a second modification of the fourth embodiment. The power supply apparatus 2C shown in Fig. 14 further includes a second high frequency power generating section 32b as a high frequency power generating section for wire breakage detection, an output transformer 35b, a second high frequency current measuring section 37b, a second high frequency voltage measuring section 39b and a band pass filter (BPF) 71, in the power supply apparatus 2 of Fig. 2.

The high frequency power generating section 32 as the high frequency power generating section for treatment generates high frequency power of a frequency f1 (an angular frequency $\omega 1$), and the second high frequency power generating section 32b generates high frequency power of a frequency f2 (an angular frequency $\omega 2$). Note that f1 > f2 is set.

A high frequency signal of the frequency f2 by the second high frequency power generating section 32b passes through the second high frequency current measuring section 37b from the secondary side that is insulated by the output transformer 35b, further passes through the band pass filter 71 and is superimposed on the high frequency signal of the frequency f1 for treatment to be outputted to the high frequency signal lines 3a and 3b side from the high frequency output terminal.

Further, the high frequency current and the high frequency voltage that are respectively measured by the second high frequency current measuring section 37b and the second high frequency voltage measuring section 39b are inputted into the control section 42 via the A/D converter 41. (The calculation section 42c inside) the control section 42 calculates impedance Zm2 by dividing the measured high frequency voltage by the high frequency current.

Note that (the determination section 42d inside) the control section 42 determines occurrence of wire breakage by comparing the impedance Zm2 that is measured in the frequency f2, and a threshold value Zth2 of the impedance that is set in advance by the threshold value setting section 48 to detect occurrence of wire breakage.

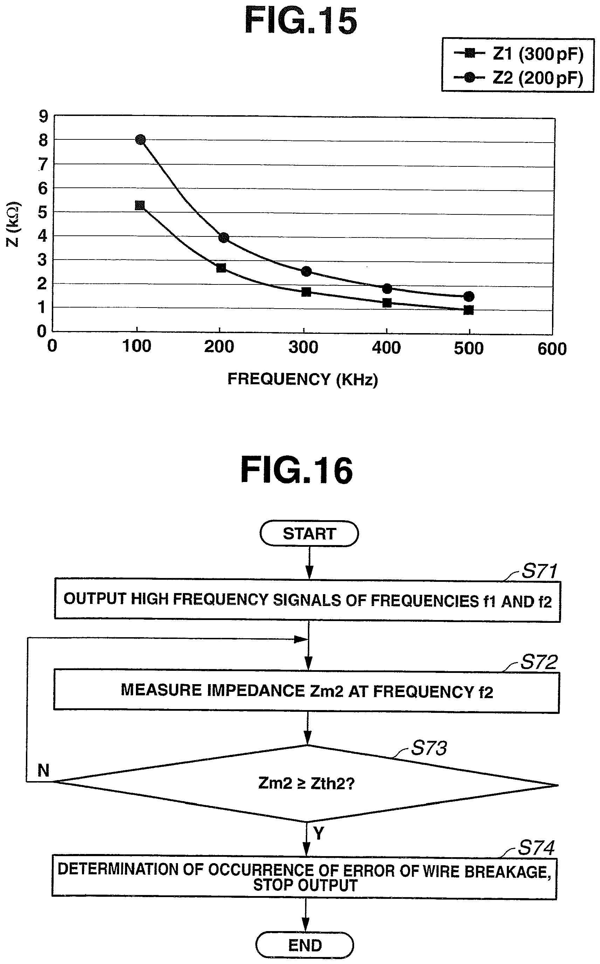

Fig. 15 shows characteristic examples of impedance values that are measured when a frequency is changed, for example, when the electrostatic capacity value of the treatment instrument 3 is 300 pF and when the electrostatic value of the treatment instrument 3 is 200 pF.

As is understandable from the characteristics of Fig. 15, when the electrostatic capacity value at the treatment instrument 3 side is measured as an impedance value, the impedance value can be measured as a larger value when the impedance value is measured at a lower frequency.

Therefore, in a surgical operation system including the power supply apparatus 2C of Fig. 14, a high frequency signal for treatment and a high frequency signal for wire detection are enabled to be simultaneously outputted. In this case, power of the high frequency signal for wire detection is set at a sufficiently small value (for example, 1 W or less) as compared with the power of the high frequency signal for treatment, and therefore, output control in a case of treatment being performed by the power of the high frequency signal for treatment can be performed by the power of the high frequency signal for wire detection being ignored.

Fig. 16 shows a flowchart of detecting occurrence of wire breakage by outputting two high frequency signals for treatment and for wire breakage detection.

When the output switch 24 is turned on in the first step S71, the control section 42 outputs the high frequency signal of the frequency f1 for treatment and the high frequency signal of the frequency f2 for wire detection to the treatment instrument 3 side.

In the next step S72, the control section 42 measures (calculates) the impedance Zm2 between the high frequency signal lines 3a and 3b of the treatment instrument 3 from the high frequency current and the high frequency voltage that are measured by the second high frequency current measuring section 37b and the second high frequency voltage measuring section 39b.

In the next step S73, the control section 42 performs determination of whether or not the measured impedance Zm2 satisfies the condition of Zm2 ≥ Zth23. When the impedance Zm2 does not satisfy the condition of Zm2 ≥ Zth2, the control section 42 returns to the processing of S72.

When the impedance Zm2 satisfies the condition of Zm2 ≥ Zth2, the control section 42 proceeds to processing of step S74. In step S74, the control section 42 determines that an error of wire breakage occurs, stops output, and ends the processing of Fig. 16. As shown in Fig. 16, the impedance Zm2 is measured with use of the frequency f2 that is lower than the frequency f1 at which treatment is performed, whereby the impedance Zm2 can be measured with higher precision, and accordingly, presence or absence of wire breakage can be determined (detected) with higher precision.

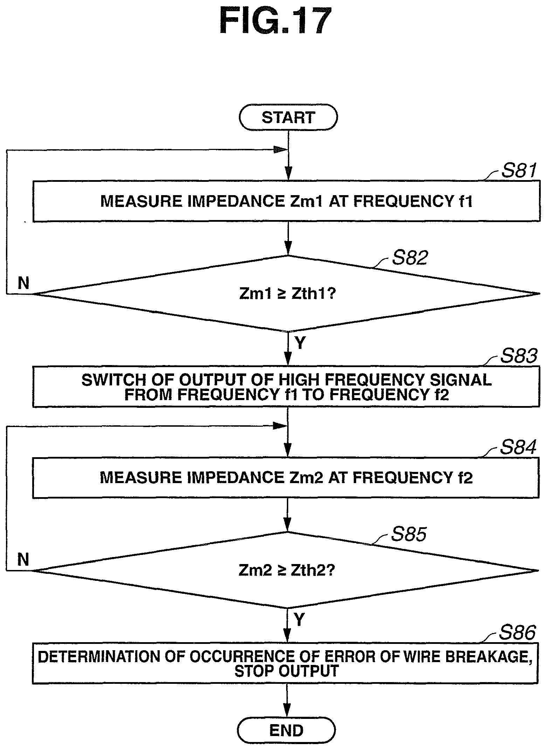

Note that in place of the processing shown in Fig. 16 being performed, occurrence of wire breakage may be detected by processing shown in Fig. 17.

When treatment is started as shown in Fig. 17, in the first step S81, the control section 42 measures impedance Zm1 from the high frequency voltage and the high frequency current in the case of the frequency f1.

In the next step S82, the determination section 42d determines occurrence of wire breakage by comparing the measured impedance Zm1 with a threshold value Zth1 of impedance that is set in advance to detect wire breakage (for example, performs comparison of whether or not Zm1 ≥ Zth1 is satisfied). When the impedance Zm1 does not satisfy the condition of Zm ≥ Zth1, the determination section 42d determines that wire breakage does not take place and returns to the processing of step S81.

When the impedance Zm1 satisfies the condition of Zm1 ≥ Zth1, the determination section 42d determines that there is a possibility of wire breakage, and proceeds to processing of step S83. In step S83, the control section 42 performs setting (control) so that, for example, the high frequency power generating section 32 does not output a high frequency signal, and performs setting (control) so that the high frequency power generating section 32b outputs the high frequency signal of the second frequency f2.

In the next step S84, the control section 42 measures the impedance Zm2 from the high frequency voltage and the high frequency current in the case of the frequency f2.

In the next step S85, (the determination section 42d of) the control section 42 determines occurrence of wire breakage by comparing the measured impedance Zm2 with the threshold value Zth2 of impedance that is set in advance to detect wire breakage (for example, performs comparison of whether or not Zm2 ≥ Zth2 is satisfied). When the impedance Zm2 does not satisfy the condition of Zm2 ≥ Zth2, the control section 42 determines that wire breakage is absent, and returns to the processing of step S84.

When the impedance Zm2 satisfies the condition of Zm2 ≥ Zth2, the control section 42 determines an error of wire breakage occurs in step S86, stops output of the high frequency signal and the ultrasound signal, and ends the processing of Fig. 17. As shown in Fig. 17, occurrence of wire breakage is determined with use of the two frequencies, whereby determination with higher reliability than in the case in which occurrence of wire breakage is determined with use of one frequency can be performed.

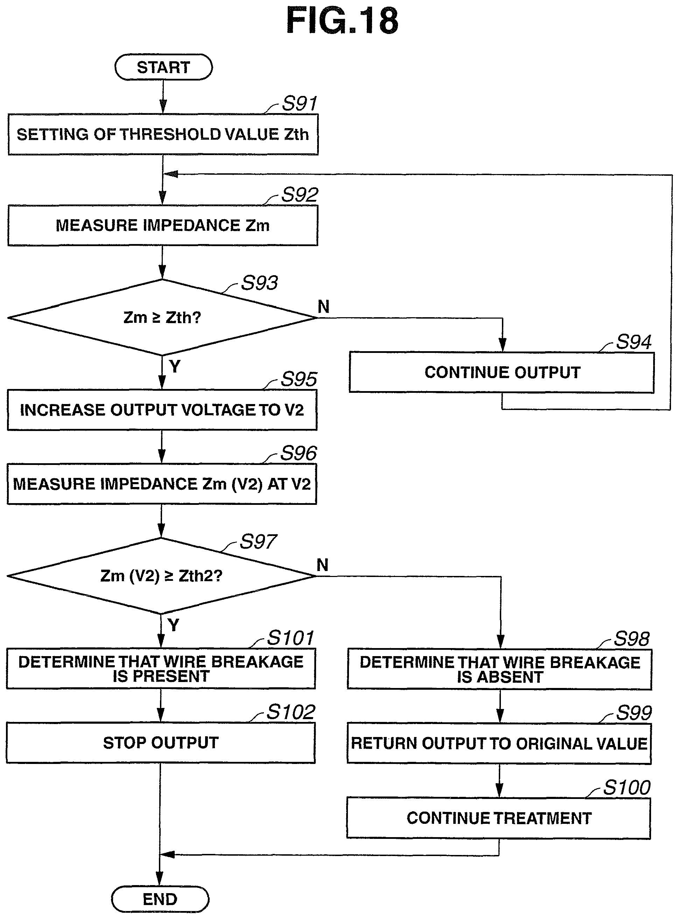

Note that, for example, when measurement of the impedance Zm1 or Zm2 is performed with use of the high frequency signal of the aforementioned frequency f1 or f2, the impedance Zm1 or Zm2 is compared with the threshold value Zth1 or Zth2, and occurrence of wire breakage is determined, occurrence of wire breakage may be determined with a processing procedure as shown in Fig. 18. In the following explanation, explanation will be performed with the measured impedance set as Zm, and the threshold value set as Zth.

In the first step S91, output of the high frequency signal and the ultrasound drive signal is started. In the next step S92, (the calculation section 42c of) the control section 42 performs measurement of the impedance Zm with use of the high frequency signal of the frequency f2, for example.

In the next step S93, the determination section 42d compares the impedance Zm and the threshold value Zth, and performs determination of whether or not the impedance Zm satisfies the condition of Zm ≥ Zth. When the impedance Zm does not satisfy the condition of Zm ≥ Zth, the determination section 42d determines that wire breakage does not occur, and in the next step S94, the control section 42 performs control so that output of the high frequency signal and the ultrasound drive signal is continued, and returns to the processing of step S92.

When the impedance Zm satisfies the condition of Zm ≥ Zth in step S93, the determination section 42d determines that there is a possibility of occurrence of wire breakage, and in step S95, the control section 42 increases the output voltage of the high frequency signal to V2. Thereafter, in the next step S96, (the calculation section 42c of) the control section 42 performs measurement of impedance (the impedance is expressed by Zm(V2)) with use of the high frequency signal of the frequency f2 and an output voltage of V2.

In the next step S97, the determination section 42d compares the impedance Zm(V2) and the threshold value Zth, and performs determination of whether or not the impedance Zm(V2) satisfies a condition of Zm(V2) ≥ Zth. When the impedance Zm(V2) does not satisfy the condition of Zm ≥ Zth, the determination section 42d determines that wire breakage does not occur as shown in step S98, and in the next step S99, the control section 42 returns the output voltage of the high frequency signal to the original value. Subsequently, the control section 42 continues treatment as shown in step S100.

When the impedance Zm(V2) satisfies the condition of Zm ≥ Zth in step S97, the determination section 42d determines that wire breakage occurs as shown in step S101, and in the next step S102, the control section 42 stops output of the high frequency signal and the ultrasound drive signal, and ends the processing of Fig. 18.

The processing procedure shown in Fig. 18 is performed, whereby even if it is determined that wire breakage occurs with the high frequency signal with a small output voltage, it is determined whether or not wire breakage occurs in the state of the high frequency signal of the output voltage V2 that is more increased, and thereby occurrence of wire breakage can be determined in a state with higher reliability.

Subsequently, on the basis of the determination result in the state of the high frequency signal of the more increased output voltage V2, control of continuation of treatment or stop of output is performed.

Note that the case of the frequency of the high frequency signal being f2 is described, but the present embodiment also can be applied to the case of the frequency of the high frequency signal being f1.

Further, in the aforementioned embodiment, the case of ultrasound energy is described as an example of other energy different from high frequency energy, but other energy different from ultrasound energy, for example, thermal energy may be adopted.

Further, embodiments that are configured by partially combining the first to the fourth embodiments (also including modifications) described above and the like also belong to the present invention. Further, the present invention is not limited to the configuration including all the components disclosed in the description and the drawings, and the case of the configuration to which a singular or a plurality of components are properly added with the configuration disclosed in claim 1 as the basis also belongs to the present invention.

**Claims**

1. A surgical operation system, comprising:

   a power supply apparatus (2) including a first signal producing section that is adapted to produce a first signal that outputs high frequency energy, and a second signal producing section that is adapted to produce a second signal that outputs other energy different from the high frequency energy;
   a surgical treatment instrument (3) that has a converter that is adapted to produce the other energy by supply of the second signal, and grasping members that are adapted to openably and closably grasp a living tissue of an object to be treated, the surgical treatment instrument (3) being adapted to perform treatment for the living tissue by the high frequency energy via a pair of electrodes that are formed by the grasping members and treatment by the other energy for the living tissue via the grasping members;
   a cable (7) that has one end connected to the surgical treatment instrument (3), and is adapted to supply the first signal and the second signal to the surgical treatment instrument (3) from the power supply apparatus (2);
   a high frequency signal line comprising a first high frequency signal line (8a, 8b, (7a, 7b, 23a, 23b)) that is inserted through an inside of the cable (7) from the power supply apparatus (2), and a second high frequency signal line (9a, 9b (23a, 23b, 18a, 18b)) that is connected to the first high frequency signal line (8a, 8b, (7a, 7b, 23a, 23b)), is inserted through an inside of the surgical treatment instrument (3), and electrically continues to the pair of electrodes;
   a high frequency voltage measuring section (39) that is provided in the power supply apparatus (2), and is adapted to measure a high frequency voltage of the first signal;
   a high frequency current measuring section (37) that is provided in the power supply apparatus (2), and is adapted to measure a high frequency current of the first signal;

a calculation section (42c) that is provided in the power supply apparatus (2) and is adapted to calculate an electrostatic capacity value (Cm) corresponding to a length of the high frequency signal line in which the high frequency current passes, on a basis of the high frequency voltage measured by the high frequency voltage measuring section (39) and the high frequency current measured by the high frequency current measuring section (37), in a middle of treating the living tissue by simultaneous output of the high frequency energy and the other energy;

a determination section (42d) that is provided in the power supply apparatus (2), and is adapted to determine whether or not the electrostatic capacity value (Cm) calculated by the calculation section (42c) is smaller than a threshold value (Cth) set in advance to detect occurrence of wire breakage in the high frequency signal line; and

a control section (42) that is provided in the power supply apparatus (2), adapted to perform control of stopping output of the first signal and the second signal that are supplied to the surgical treatment instrument (3) via the cable (7) from the power supply apparatus (2), and is adapted to cause a display section (49) to display occurrence of the wire breakage in the high frequency signal line and stoppage of the output of the first signal and the second signal when the determination section (42d) determines that the electrostatic capacity value (Cm) is smaller than the threshold value (Cth).

2. The surgical operation system according to claim 1, further comprising:

a threshold value setting section (48) that is adapted to set the threshold value (Cth), wherein the threshold value setting section (48) sets the threshold value (Cth) at a value smaller than a first electrostatic capacity value that is calculated or measured in advance for the surgical treatment instrument (3) to which the cable (7) is connected in a state in which the high frequency signal line does not have wire breakage.

3. The surgical operation system according to claim 1, wherein the surgical treatment instrument (3) is a high frequency/ultrasound treatment instrument that has an ultrasound transducer that is adapted to perform ultrasound vibration as the converter by being supplied with the second signal, and has an ultrasound vibration transmitting rod that is adapted to transmit the ultrasound vibration generated by the ultrasound transducer to one of the pair of electrodes that are formed by the grasping members.

4. The surgical operation system according to claim 3, wherein the high frequency/ultrasound treatment instrument is configured by an ultrasound transducer unit including the ultrasound transducer to which the cable (7) is connected, and a handle unit including a fitting portion to which a front end side of the ultrasound transducer unit is adapted to be detachably fitted, and

the second high frequency signal line is configured by a third high frequency signal line disposed inside the ultrasound transducer unit, and a fourth high frequency signal line inside the handle unit that is connected to the third high frequency signal line via a contact point provided in the fitting portion, and is adapted to transmit the high frequency signal to the pair of electrodes.

5. The surgical operation system according to claim 4, wherein in response to a case in which the handle unit is replaced with new one each time treatment is performed, the threshold value setting section (48) is adapted to set, as the threshold value, a second threshold value that is slightly smaller than a second electrostatic capacity value that is calculated or measured in advance for the ultrasound transducer unit to which the cable (7) is connected in a state in which the first high frequency signal line inside the cable (7) and the third high frequency signal line do not have wire breakage.

6. The surgical operation system according to claim 5, wherein the second threshold value is set within a range of 150 pF to 250 pF.

7. The surgical operation system according to claim 4, wherein the threshold value setting section (48) is adapted to set the second threshold value on a basis of a second electrostatic capacity value that is calculated by the calculation section (42c) when a connector provided at the other end of the cable (7) is connected to the power supply apparatus (2) and is calculated or measured in a state in which the first high frequency signal line and the third high frequency signal line do not have wire breakage, for the ultrasound transducer unit to which the cable (7) is connected in a state in which the handle unit is not fitted.

8. The surgical operation system according to claim 5, further comprising:

a third signal generating section that is adapted to generate a third signal of a frequency lower than a frequency of the first signal; and

a second high frequency voltage measuring section and a second high frequency current measuring section that are respectively adapted to measure a second high frequency voltage and a second high frequency current of the third signal that is outputted to the high frequency/ultrasound treatment instrument side via the cable (7),

wherein the calculation section (42c) is adapted to calculate a third electrostatic capacity value of the high frequency/ultrasound treatment instrument to which the cable (7) is connected from the second high frequency voltage and the second high frequency current that are measured, and

the determination section (42d) is adapted to determine whether or not the third electrostatic capacity value that is calculated by the calculation section (42c) is smaller than the threshold value that is set in advance to detect occurrence of wire breakage.

9. The surgical operation system according to claim 5, further comprising:

a display section (49) that is adapted to display occurrence of the wire breakage in the high frequency signal line and stoppage of the output of the first signal and the second signal, as a determination result by the determination section (42d).

10. The surgical operation system according to claim 9, wherein the high frequency/ultrasound treatment instrument has an information storage section that is adapted to store information of an electrostatic capacity value that the high frequency/ultrasound treatment instrument has in a state in which the first high frequency signal line of the cable (7) connected to the high frequency/ultrasound treatment instrument and the second high frequency signal line inside the high frequency/ultrasound treatment instrument do not have wire breakage.

11. The surgical operation system according to claim 10, wherein the threshold value setting section (48) is adapted to set the threshold value on a basis of the information stored in the information storage section.

12. The surgical operation system according to claim 5, further comprising:

a storage section that is adapted to store the electrostatic capacity value calculated by the calculation section (42c) for the high frequency/ultrasound treatment instrument that is connected to the cable (7), in time sequence at every predetermined time; and

a second determination section that is adapted to determine whether or not an absolute value of a difference value of two electrostatic capacity values that are a present electrostatic capacity value that is calculated by the calculation section (42c) and a past electrostatic capacity value that is calculated a predetermined time before and is stored in the storage section is less than a third threshold value that is set in response to the predetermined time,

wherein when the second determination section determines that the absolute value of the difference value is the third threshold value or more, the control section (42) is adapted to perform control of stopping output of the high frequency signal and the second signal that are supplied from the power supply apparatus to the surgical treatment instrument via the cable (7).

13. The surgical operation system according to claim 12, wherein the third threshold value is set at a value of 1/10 of the second threshold value or less.

**Patentansprüche**

1. Chirurgisches Operationssystem, umfassend:

ein Energieversorgungsgerät (2), das einen ersten Signalerzeugungsabschnitt, der geeignet ist, um ein erstes Signal, das Hochfrequenzenergie ausgibt, zu erzeugen, und einen zweiten Signalerzeugungsabschnitt, der geeignet ist, um ein zweites Signal, das eine andere Energie als die Hochfrequenzenergie ausgibt, zu erzeugen, umfasst;

ein chirurgisches Behandlungsinstrument (3), das einen Umrichter, der geeignet ist, um die andere Energie zu erzeugen, indem er das zweite Signal liefert, und Greifelemente, die geeignet sind, um durch Öffnen und Schließen ein lebendes Gewebe eines zu behandelnden Objekts zu ergreifen, aufweist, wobei das chirurgische Behandlungsinstrument (3) geeignet ist, um eine Behandlung für das lebende Gewebe durch die Hochfrequenzenergie anhand eines Elektrodenpaars, das durch die Greifelemente gebildet wird, und eine Behandlung durch die andere Energie für das lebende Gewebe anhand der Greifelemente auszuführen;

ein Kabel (7), dessen eines Ende mit dem chirurgischen Behandlungsinstrument (3) verbunden ist und das geeignet ist, um das erste Signal und das zweite Signal an das chirurgische Behandlungsinstrument (3) von dem Energieversorgungsgerät (2) zu liefern;

eine Hochfrequenz-Signalleitung, die eine erste Hochfrequenz-Signalleitung (8a, 8b, (7a, 7b, 23a, 23b)), die durch das Innere des Kabels (7) von dem Energieversorgungsgerät (2) aus eingefügt wird, umfasst, und eine zweite Hochfrequenz-Signalleitung (9a, 9b (23a, 23b, 18a, 18b)), die mit der ersten Hochfrequenz-Signalleitung (8a, 8b, (7a, 7b, 23a, 23b)) verbunden ist, wird durch das Innere des chirurgischen Behandlungsinstruments (3) eingefügt und geht elektrisch bis zu dem Elektrodenpaar weiter; einen Hochfrequenz-Spannungsmessabschnitt (39), der in dem Energieversorgungsgerät (2) bereitgestellt wird und geeignet ist, um eine Hochfrequenz-Spannung des ersten Signals zu messen; einen Hochfrequenz-Strommessabschnitt (37), der in dem Energieversorgungsgerät (2) bereitgestellt wird und geeignet ist, um einen Hochfrequenz-Strom des ersten Signals zu messen; einen Berechnungsabschnitt (42c), der in dem Energieversorgungsgerät (2) bereitgestellt wird und geeignet ist, um einen elektrostatischen Kapazitätswert (Cm), der einer Länge der Hochfrequenz-Signalleitung entspricht, in welcher der Hochfrequenz-Strom fließt, auf der Grundlage der Hochfrequenz-Spannung, die von dem Hochfrequenz-Spannungsmessabschnitt (39) gemessen wird, und des Hochfrequenz-Stroms, der von dem Hochfrequenz-Strommessabschnitt (37) gemessen wird, während der Behandlung des lebenden Gewebes durch die gleichzeitige Ausgabe der Hochfrequenz-Energie und der anderen Energie zu berechnen; einen Bestimmungsabschnitt (42d), der in dem Energieversorgungsgerät (2) bereitgestellt wird und geeignet ist, um zu bestimmen, ob der elektrostatische Kapazitätswert (Cm), der von dem Berechnungsabschnitt (42c) berechnet wird, kleiner als ein Schwellenwert (Cth), der im Voraus eingestellt wird, ist oder nicht, um das Vorkommen eines Drahtbruchs in der Hochfrequenz-Signalleitung zu detektieren; und einen Steuerabschnitt (42), der in dem Energieversorgungsgerät (2) bereitgestellt wird, der geeignet ist, eine Steuerung eines Stoppens der Ausgabe des ersten Signals und des zweiten Signals, die dem chirurgischen Behandlungsinstrument (3) über das Kabel (7) von dem Energieversorgungsgerät (2) aus geliefert werden, auszuführen, und der geeignet ist, zu bewirken, dass ein Anzeigeabschnitt (49) das Vorkommen des Drahtbruchs in der Hochfrequenz-Signalleitung und des Stoppens der Ausgabe des ersten Signals und des zweiten Signals anzuzeigen, wenn der Bestimmungsabschnitt (42d) bestimmt, dass der elektrostatische Kapazitätswert (Cm) kleiner als der Schwellenwert (Cth ist).

2. Chirurgisches Operationssystem nach Anspruch 1, ferner umfassend:

   einen Abschnitt (48) zum Einstellen von Schwellenwerten, der geeignet ist, um den Schwellenwert (Cth) einzustellen, wobei der Abschnitt (48) zum Einstellen von Schwellenwerten den Schwellenwert (Cth) auf einen Wert einstellt, der kleiner als ein erster elektrostatischer Kapazitätswert ist, der für das chirurgische Behandlungsinstrument (3), mit dem das Kabel (7) verbunden ist, in einem Zustand, in dem die Hochfrequenz-Signalleitung keinen Drahtbruch aufweist, im Voraus berechnet oder gemessen wird.

3. Chirurgisches Operationssystem nach Anspruch 1, wobei das chirurgische Behandlungsinstrument (3) ein Hochfrequenz-/ Ultraschall-Behandlungsinstrument ist, das einen Ultraschallwandler aufweist, der geeignet ist, um eine Ultraschallschwingung als Umrichter auszuführen, indem er mit dem zweiten Signal versorgt wird, und einen Stab zum Übertragen von Ultraschallschwingungen aufweist, der geeignet ist, um die Ultraschallvibration, die durch den Ultraschallwandler generiert wird, auf eine von dem Elektrodenpaar, das durch die Greifelemente gebildet wird, zu übertragen.

4. Chirurgisches Operationssystem nach Anspruch 3, wobei das Hochfrequenz-/ Ultraschall-Behandlungsinstrument durch eine Ultraschallwandlereinheit, die den Ultraschallwandler umfasst, mit dem das Kabel (7) verbunden ist, und eine Griffeinheit, die einen Montageteil umfasst, an den eine Frontseite der Ultraschallwandlereinheit abnehmbar montiert werden kann, konfiguriert ist, und die zweite Hochfrequenz-Signalleitung durch eine dritte Hochfrequenz-Signalleitung, die im Innern der Ultraschall-Umrichtereinheit angeordnet ist, und eine vierte Hochfrequenz-Signalleitung im Innern der Griffeinheit, die mit der dritten Hochfrequenz-Signalleitung über einen Kontaktpunkt, der in dem Montageteil bereitgestellt ist, konfiguriert ist und geeignet ist, um das Hochfrequenz-Signal an das Elektrodenpaar zu senden.

5. Chirurgisches Operationssystem nach Anspruch 4, wobei als Reaktion auf den Fall, dass die Griffeinheit jedes Mal, wenn eine Behandlung erfolgt, durch eine neue ersetzt wird, der Abschnitt (48) zum Einstellen von Schwellenwerten geeignet ist, um als Schwellenwert einen zweiten Schwellenwert einzustellen, der etwas kleiner als ein zweiter elektrostatischer Kapazitätswert ist, der für die Ultraschallwandlereinheit, mit der das Kabel (7) verbunden ist, in einem Zustand, in dem die erste Hochfrequenz-Signalleitung im Innern des Kabels (7) und die dritte Hochfrequenz-Signalleitung keinen Drahtbruch aufweisen, im Voraus berechnet oder gemessen wird.

6. Chirurgisches Operationssystem nach Anspruch 5, wobei der zweite Schwellenwert in einem Bereich von 150 pF bis 250 pF eingestellt wird.

7. Chirurgisches Operationssystem nach Anspruch 4, wobei der Abschnitt (48) zum Einstellen von Schwellenwerten geeignet ist, um den zweiten Schwellenwert auf der Grundlage eines zweiten elektrostatischen Kapazitätswerts einzustellen, der von dem Berechnungsabschnitt (42c) berechnet wird, wenn ein Verbindungsteil, das an dem anderen Ende des Kabels (7) bereitgestellt ist, mit dem Energieversorgungsgerät (2) verbunden ist, und in einem Zustand, in dem die erste Hochfrequenz-Signalleitung und die dritte Hochfrequenz-Signalleitung keinen Drahtbruch aufweisen, für die Ultraschallwandlereinheit, mit der das Kabel (7) verbunden ist, in einem Zustand, in dem der Griff nicht montiert ist, berechnet oder gemessen wird.

8. Chirurgisches Operationssystem nach Anspruch 5, ferner umfassend:

   einen dritten Signalgenerierungsabschnitt, der geeignet ist, um ein drittes Signal einer Frequenz zu generieren, die niedriger als eine Frequenz des ersten Signals ist; und
   einen zweiten Hochfrequenz-Spannungsmessabschnitt und einen zweiten Hochfrequenz-Strommessabschnitt, die jeweils geeignet sind, um eine zweite Hochfrequenz-Spannung und einen zweiten Hochfrequenz-Strom des dritten Signals zu messen, das an die Seite des Hochfrequenz-/ Ultraschall-Behandlungsinstruments über das Kabel (7) ausgegeben wird,
   wobei der Berechnungsabschnitt (42c) geeignet ist, um einen dritten elektrostatischen Kapazitätswert des Hochfrequenz-/ Ultraschall-Behandlungsinstruments, mit dem das Kabel (7) verbunden ist, aus der zweiten Hochfrequenz-Spannung und dem zweiten Hochfrequenz-Strom, die gemessen werden, zu berechnen, und
   der Bestimmungsabschnitt (42d) geeignet ist, um zu bestimmen, ob der dritte elektrostatische Kapazitätswert, der von dem Berechnungsabschnitt (42c) berechnet wird, kleiner als der Schwellenwert, der im Voraus eingestellt wird, ist oder nicht, um das Vorkommen eines Drahtbruchs zu detektieren.

9. Chirurgisches Operationssystem nach Anspruch 5, ferner umfassend:

   einen Anzeigeabschnitt (49), der geeignet ist, um das Vorkommen des Drahtbruchs in der Hochfrequenz-Signalleitung und das Stoppens der Ausgabe des ersten Signals und des zweiten Signals als Bestimmungsergebnis durch den Bestimmungsabschnitt (42d) anzuzeigen.

10. Chirurgisches Operationssystem nach Anspruch 9, wobei das Hochfrequenz-/ Ultraschall-Behandlungsinstrument einen Informationsspeicherabschnitt aufweist, der geeignet ist, um Informationen eines elektrostatischen Kapazitätswerts zu speichern, die das Hochfrequenz-/ Ultraschall-Behandlungsinstrument in einem Zustand aufweist, in dem die erste Hochfrequenz-Signalleitung des Kabels (7), die mit dem Hochfrequenz-/ Ultraschall-Behandlungsinstrument verbunden ist, und die zweite Hochfrequenz-Signalleitung im Innern des Hochfrequenz-/ Ultraschall-Behandlungsinstruments keinen Drahtbruch aufweisen.

11. Chirurgisches Operationssystem nach Anspruch 10, wobei der Abschnitt (48) zum Einstellen von Schwellenwerten geeignet ist, um den Schwellenwert auf der Grundlage der Informationen, die in dem Informationsspeicherabschnitt gespeichert sind, einzustellen.

12. Chirurgisches Operationssystem nach Anspruch 5, ferner umfassend:

   einen Speicherabschnitt, der geeignet ist, um den elektrostatischen Kapazitätswert, der von dem Berechnungsabschnitt (42c) für das Hochfrequenz-/ Ultraschall-Behandlungsinstrument, das mit dem Kabel (7) verbunden ist, berechnet wird, in einer Zeitsequenz zu jedem vorbestimmten Zeitpunkt zu speichern; und
   einen zweiten Bestimmungsabschnitt, der geeignet ist, um zu bestimmen, ob ein absoluter Wert eines Differenzwerts von zwei elektrostatischen Kapazitätswerten, wobei es sich um einen vorliegenden elektrostatischen Kapazitätswert, der durch den Berechnungsabschnitt (42c) berechnet wird, und einen vergangenen elektrostatischen Kapazitätswert, der zu einem vorbestimmten Zeitpunkt zuvor berechnet wurde und in dem Speicherabschnitt gespeichert ist, handelt, kleiner als ein dritter Schwellenwert, der als Reaktion auf den vorbestimmten Zeitpunkt eingestellt wird, ist oder nicht,
   wobei, wenn der zweite Bestimmungsabschnitt bestimmt, dass der absolute Wert des Differenzwerts der dritte Schwellenwert oder mehr ist, der Steuerabschnitt (42) geeignet ist, um eine Steuerung eines Stoppens der Ausgabe des HochfrequenzSignals und des zweiten Signals, die von dem Stromversorgungsgerät an das chirurgische Behandlungsinstrument über das Kabel (7) geliefert werden, auszuführen.

13. Chirurgisches Operationssystem nach Anspruch 12, wobei der dritte Schwellenwert auf einen Wert von 1/10 des zweiten Schwellenwerts oder weniger eingestellt ist.

## Revendications

1. Système d'opération chirurgicale, comprenant :

un appareil d'alimentation électrique (2) comprenant une première section de production de signal qui est adaptée pour produire un premier signal qui délivre une énergie à haute fréquence, et une deuxième section de production de signal qui est adaptée pour produire un deuxième signal qui délivre une autre énergie différente de l'énergie à haute fréquence ;

un instrument de traitement chirurgical (3) qui a un convertisseur qui est adapté pour produire l'autre énergie par fourniture du deuxième signal, et des éléments de saisie qui sont adaptés pour saisir par ouverture et fermeture un tissu vivant d'un sujet à traiter, l'instrument de traitement chirurgical (3) étant adapté pour réaliser un traitement pour le tissu vivant par l'énergie à haute fréquence par l'intermédiaire d'une paire d'électrodes qui sont formées par les éléments de saisie et un traitement par l'autre énergie pour le tissu vivant par l'intermédiaire des éléments de saisie ;

un câble (7) qui a une extrémité reliée à l'instrument de traitement chirurgical (3), et qui est adapté pour fournir le premier signal et le deuxième signal à l'instrument de traitement chirurgical (3) à partir de l'appareil d'alimentation électrique (2) ;

une ligne de signal à haute fréquence comprenant une première ligne de signal à haute fréquence (8a, 8b, (7a, 7b, 23a, 23b)) qui est insérée à travers un intérieur du câble (7) à partir de l'appareil d'alimentation électrique (2), et une deuxième ligne de signal à haute fréquence (9a, 9b (23a, 23b, 18a, 18b)) qui est reliée à la première ligne de signal à haute fréquence (8a, 8b, (7a, 7b, 23a, 23b)), est insérée à travers un intérieur de l'instrument de traitement chirurgical (3), et continuant la liaison électrique jusqu'à la paire d'électrodes ;

une section de mesure de tension à haute fréquence (39) qui est disposée dans l'appareil d'alimentation électrique (2) et qui est adaptée pour mesurer une tension à haute fréquence du premier signal ;

une section de mesure de courant à haute fréquence (37) qui est disposée dans l'appareil d'alimentation électrique (2), et qui est adaptée pour mesurer un courant à haute fréquence du premier signal ;

une section de calcul (42c) qui est disposée dans l'appareil d'alimentation électrique (2) et qui est adaptée pour calculer une valeur de capacité électrostatique (Cm) correspondant à une longueur de la ligne de signal à haute fréquence dans laquelle le courant à haute fréquence passe, sur une base de la tension à haute fréquence mesurée par la section de mesure de tension à haute fréquence (39) et du courant à haute fréquence mesuré par la section de mesure de courant à haute fréquence (37), au milieu du traitement du tissu vivant, par délivrance simultanée de l'énergie à haute fréquence et de l'autre énergie ;

une section de détermination (42d) qui est disposée dans l'appareil d'alimentation électrique (2), et qui est adaptée pour déterminer si ou non la valeur de capacité électrostatique (Cm) calculée par la section de calcul (42c) est plus petite qu'une valeur de seuil (Cth) réglée à l'avance pour détecter l'occurrence d'une rupture de fil dans la ligne de signal à haute fréquence ; et

une section de commande (42) qui est disposée dans l'appareil d'alimentation électrique (2), qui est adaptée pour réaliser la commande de l'arrêt de la délivrance du premier signal et du deuxième signal qui sont fournis à l'instrument de traitement chirurgical (3) par l'intermédiaire du câble (7) à partir de l'appareil d'alimentation électrique (2), et qui est adaptée pour amener une section d'affichage (49) à afficher l'occurrence de la rupture de fil dans la ligne de signal à haute fréquence et l'arrêt de la délivrance du premier signal et du deuxième signal lorsque la section de détermination (42d) détermine que la valeur de capacité électrostatique (Cm) est plus petite que la valeur de seuil (Cth).

2. Système d'opération chirurgicale selon la revendication 1, comprenant en outre :

une section de réglage de valeur de seuil (48) qui est adaptée pour régler la valeur de seuil (Cth),

la section de réglage de valeur de seuil (48) réglant la valeur de seuil (Cth) à une valeur plus petite qu'une première valeur de capacité électrostatique qui est calculée ou mesurée à l'avance pour l'instrument de traitement chirurgical (3) auquel le câble (7) est relié dans un état dans lequel la ligne de signal à haute fréquence n'a pas de rupture de fil.

3. Système d'opération chirurgicale selon la revendication 1,
dans lequel l'instrument de traitement chirurgical (3) est un instrument de traitement à haute fréquence/ultrasons qui a, en tant que convertisseur, un transducteur à ultrasons qui est adapté pour réaliser une vibration ultrasonore en étant alimenté avec le deuxième signal, et a une tige de transmission de vibration ultrasonore qui est adaptée pour transmettre la vibration ultrasonore générée par le transducteur à ultrasons à l'une de la paire d'électrodes qui sont formées par les éléments de saisie.

4. Système d'opération chirurgicale selon la revendication 3,

   dans lequel l'instrument de traitement à haute fréquence/ultrasons est configuré par une unité de transducteur à ultrasons comprenant le transducteur à ultrasons auquel le câble (7) est relié, et une unité de poignée comprenant une partie d'adaptation à laquelle un côté d'extrémité avant de l'unité de transducteur à ultrasons est apte à être adapté de manière détachable, et

   la deuxième ligne de signal à haute fréquence est configurée par une troisième ligne de signal à haute fréquence disposée à l'intérieur de l'unité de transducteur à ultrasons, et une quatrième ligne de signal à haute fréquence à l'intérieur de l'unité de poignée qui est reliée à la troisième ligne de signal à haute fréquence par l'intermédiaire d'un point de contact prévu dans la partie d'adaptation, et est adaptée pour transmettre le signal à haute fréquence à la paire d'électrodes.

5. Système d'opération chirurgicale selon la revendication 4,

   dans lequel, en réponse à un cas dans lequel l'unité de poignée est remplacée par une nouvelle à chaque fois qu'un traitement est réalisé, la section de réglage de valeur de seuil (48) est adaptée pour régler, en tant que valeur de seuil, une deuxième valeur de seuil qui est légèrement plus petite qu'une deuxième valeur de capacité électrostatique qui est calculée ou mesurée à l'avance pour l'unité de transducteur à ultrasons à laquelle le câble (7) est relié dans un état dans lequel la première ligne de signal à haute fréquence à l'intérieur du câble (7) et la troisième ligne de signal à haute fréquence n'ont pas de rupture de fil.

6. Système d'opération chirurgicale selon la revendication 5,

   dans lequel la deuxième valeur de seuil est réglée dans une plage de 150 pF à 250 pF.

7. Système d'opération chirurgicale selon la revendication 4,

   dans lequel la section de réglage de valeur de seuil (48) est adaptée pour régler la deuxième valeur de seuil sur une base d'une deuxième valeur de capacité électrostatique qui est calculée par la section de calcul (42c) lorsqu'un connecteur prévu à l'autre extrémité du câble (7) est relié à l'appareil d'alimentation électrique (2) et est calculée ou mesurée dans un état dans lequel la première ligne de signal à haute fréquence et la troisième ligne de signal à haute fréquence n'ont pas de rupture de fil, pour l'unité de transducteur à ultrasons à laquelle le câble (7) est relié dans un état dans lequel l'unité de poignée n'est pas adaptée.

8. Système d'opération chirurgicale selon la revendication 5, comprenant en outre :

   une troisième section de génération de signal qui est adaptée pour générer un troisième signal de fréquence plus petite qu'une fréquence du premier signal ; et
   une seconde section de mesure de tension à haute fréquence et une seconde section de mesure de courant à haute fréquence qui sont adaptées pour mesurer respectivement une seconde tension à haute fréquence et un second courant à haute fréquence du troisième signal qui est délivré à l'instrument de traitement à haute fréquence/ultrasons par l'intermédiaire du câble (7),
   la section de calcul (42c) étant adaptée pour calculer une troisième valeur de capacité électrostatique de l'instrument de traitement à haute fréquence/ultrasons auquel le câble (7) est relié, à partir de la seconde tension à haute fréquence et du second courant à haute fréquence qui sont mesurés, et
   la section de détermination (42d) est adaptée pour déterminer si ou non la troisième valeur de capacité électrostatique qui est calculée par la section de calcul (42c) est plus petite que la valeur de seuil qui est réglée à l'avance pour détecter l'occurrence d'une rupture de fil.

9. Système d'opération chirurgicale selon la revendication 5, comprenant en outre :

   une section d'affichage (49) qui est adaptée pour afficher l'occurrence de la rupture de fil dans la ligne de signal à haute fréquence et l'arrêt de la délivrance du premier signal et du deuxième signal, en tant que résultat de détermination de la section de détermination (42d).

10. Système d'opération chirurgicale selon la revendication 9,

    dans lequel l'instrument de traitement à haute fréquence/ultrasons a une section de stockage d'informations qui est adaptée pour stocker des informations d'une valeur de capacité électrostatique que l'instrument de traitement à haute fréquence/ultrasons a dans un état dans lequel la première ligne de signal à haute fréquence du câble (7) relié à l'instrument de traitement à haute fréquence/ultrasons et la deuxième ligne de signal à haute fréquence à l'intérieur de l'instrument de traitement à haute fréquence/ultrasons n'ont pas de rupture de fil.

11. Système d'opération chirurgicale selon la revendication 10,
dans lequel la section de réglage de valeur de seuil (48) est adaptée pour régler la valeur de seuil sur une base des informations stockées dans la section de stockage d'informations.

12. Système d'opération chirurgicale selon la revendication 5, comprenant en outre :

une section de stockage qui est adaptée pour stocker la valeur de capacité électrostatique calculée par la section de calcul (42c) pour l'instrument de traitement à haute fréquence/ultrasons qui est relié au câble (7), dans une séquence temporelle à chaque temps prédéterminé ; et
une seconde section de détermination qui est adaptée pour déterminer si ou non une valeur absolue d'une valeur de différence de deux valeurs de capacité électrostatique, qui sont une valeur de capacité électrostatique courante qui est calculée par la section de calcul (42c) et une valeur de capacité électrostatique antérieure qui est calculée à un temps antérieur prédéterminé et est stockée dans la section de stockage, est plus petite qu'une troisième valeur de seuil qui est réglée en réponse au temps prédéterminé, dans lequel, lorsque la seconde section de détermination déterminant que la valeur absolue de la valeur de différence est la troisième valeur de seuil ou plus, la section de commande (42) est adaptée pour réaliser la commande de l'arrêt de la délivrance du signal à haute fréquence et du deuxième signal qui sont fournis à partir de l'appareil d'alimentation électrique à l'instrument de traitement chirurgical par l'intermédiaire du câble (7).

13. Système d'opération chirurgicale selon la revendication 12,
dans lequel la troisième valeur de seuil est réglée à une valeur de 1/10 de la deuxième valeur de seuil ou moins.

# FIG.1

DISPLAY SECTION

# FIG.2

# FIG.3

POWER SUPPLY APPARATUS

Cu

Ch

R

5a

(3a)

3

8    9

4

2    5b

(3b)

# FIG.4

T

V

0

-V

VOLTAGE WAVEFORM

t

Δt

I

0

-I

CURRENT WAVEFORM

t

# FIG.5

Ic

I

θ

Ir

V

# FIG.6

START

INITIAL SETTING INCLUDING
SETTING OF THRESHOLD VALUE Cth — S1

IS OUTPUT SWITCH
TURNED ON? — S2  N

Y

SIMULTANEOUS OUTPUT OF HF SIGNAL
AND US DRIVE SIGNAL — S3

CONTROL OF HF OUTPUT BY
MEASUREMENT OF HF VOLTAGE
AND HF CURRENT — S4

CONTROL OF US OUTPUT BY
MEASUREMENT OF US DRIVE VOLTAGE
AND US DRIVE CURRENT — S5

CALCULATION OF ELECTROSTATIC
CAPACITY VALUE Cm OF HF SIGNAL LINE — S6

CONTINUE OUTPUT — S8

Cm < Cth? — S7  N

IS OUTPUT SWITCH
TURNED OFF? — S9  N

Y

Y

STOP OUTPUT OF HF SIGNAL AND
US DRIVE SIGNAL — S12

STOP OUTPUT OF HF SIGNAL AND
US DRIVE SIGNAL — S10

WIRE BREAKAGE DISPLAY/
DISPLAY OF STOP OF OUTPUT — S13

END TREATMENT? — S11  N

Y

END

# FIG.7A

EP 2 796 105 B1

# FIG.7B

```
                    ( START )
                        │
                        ▼                        S1
        ┌─────────────────────────────────┐
        │   INITIAL SETTING INCLUDING      │
        │  SETTING OF THRESHOLD VALUE Cth  │
        └─────────────────────────────────┘
                        │
                        ▼              S2                              S8
              ◇                                    ┌──────────────────────────┐
         IS OUTPUT SWITCH      N ──────────────►   │     CONTINUE OUTPUT       │
           TURNED ON?                               └──────────────────────────┘
              ◇                                                │           S41
                 │ Y              S3                ┌──────────────────────────┐
        ┌─────────────────────────────────┐        │       STORE Cm (j)         │
        │ SIMULTANEOUS OUTPUT OF HF SIGNAL │        └──────────────────────────┘
        │       AND US DRIVE SIGNAL        │                   │           S42
        └─────────────────────────────────┘                   ▼
                        │                              ◇                    N
                        ▼              S4                 δ ELAPSES?  ─────►
        ┌─────────────────────────────────┐          ◇
        │    CONTROL OF HF OUTPUT BY       │              │ Y
        │  MEASUREMENT OF HF VOLTAGE       │   ┌──────────────────────────┐
        │        AND HF CURRENT            │   │  CALCULATION OF Cm (j + 1)│
        └─────────────────────────────────┘   └──────────────────────────┘
                        │                                  │           S44
                        ▼              S5         ◇                        Y
        ┌─────────────────────────────────┐      |Cm (j) – Cm (j + 1)| ────►
        │    CONTROL OF US OUTPUT BY       │         < Cth (δ)?
        │MEASUREMENT OF US DRIVE VOLTAGE   │      ◇
        │      AND US DRIVE CURRENT        │         │ N            S45
        └─────────────────────────────────┘   ┌──────────────────────────┐
                        │              S6      │ STOP OUTPUT OF HF SIGNAL  │
        ┌─────────────────────────────────┐   │   AND US DRIVE SIGNAL     │
        │  CALCULATION OF ELECTROSTATIC    │   └──────────────────────────┘
        │CAPACITY VALUE Cm OF HF SIGNAL LINE│              │           S46
        └─────────────────────────────────┘   ┌──────────────────────────┐
                        │              S7      │   ABNORMALITY DISPLAY/    │
              ◇                                │ DISPLAY OF STOP OF OUTPUT │
           Cm < Cth?            N ──►          └──────────────────────────┘
              ◇                                            │
                 │ Y              S12                       ▼          S9
        ┌─────────────────────────────────┐      ◇
        │ STOP OUTPUT OF HF SIGNAL AND     │     IS OUTPUT SWITCH     N ──►
        │       US DRIVE SIGNAL            │       TURNED OFF?
        └─────────────────────────────────┘      ◇
                        │              S13           │ Y          S10
        ┌─────────────────────────────────┐   ┌──────────────────────────┐
        │    WIRE BREAKAGE DISPLAY/        │   │ STOP OUTPUT OF HF SIGNAL  │
        │  DISPLAY OF STOP OF OUTPUT       │   │   AND US DRIVE SIGNAL     │
        └─────────────────────────────────┘   └──────────────────────────┘
                        │                                  │           S11
                        │                          ◇
                        ▼                       END TREATMENT?      N ──►
                    ( END )                         │ Y
```

# FIG.8

INFORMATION STORAGE

*61*

*62*

*2B*

*3B*

*7* *12*

*8*

*9*

*1B*

# FIG.9

EP 2 796 105 B1

# FIG.10

START

S21
CONNECT TREATMENT INSTRUMENT
INCLUDING ULTRASOUND TRANSDUCER UNIT

S22
READ INFORMATION FROM
ULTRASOUND TRANSDUCER UNIT

S23
SET Cu AS THRESHOLD VALUE Cth

S2
IS OUTPUT SWITCH
TURNED ON? — N

Y

S3
SIMULTANEOUS OUTPUT OF HF SIGNAL
AND US DRIVE SIGNAL

S4
CONTROL OF HF OUTPUT BY
MEASUREMENT OF HF VOLTAGE
AND HF CURRENT

S5
CONTROL OF US OUTPUT BY
MEASUREMENT OF US DRIVE VOLTAGE
AND US DRIVE CURRENT

S6
CALCULATION OF ELECTROSTATIC
CAPACITY VALUE Cm OF HF SIGNAL LINE

S8
CONTINUE OUTPUT

S7
$Cm < Cth$? — N

S9
IS OUTPUT SWITCH
TURNED OFF? — N

Y

Y

S12
STOP OUTPUT OF HF SIGNAL AND
US DRIVE SIGNAL

S10
STOP OUTPUT OF HF SIGNAL AND
US DRIVE SIGNAL

S13
WIRE BREAKAGE DISPLAY/
DISPLAY OF STOP OF OUTPUT

S11
END TREATMENT? — N

Y

END

33

# FIG.11

```
                    ┌─────────────┐
                    │    START    │
                    └──────┬──────┘
                           │              ╭─S31
              ┌────────────▼────────────┐
              │  CONNECTION OF ULTRASOUND │
              │     TRANSDUCER UNIT       │
              └────────────┬────────────┘
                           │              ╭─S32
              ┌────────────▼────────────┐
              │    CONFIRM CONTINUITY    │
              └────────────┬────────────┘
                           │              ╭─S33
              ┌────────────▼────────────┐
              │ MEASUREMENT OF ELECTROSTATIC │
              │    CAPACITY VALUE Cf      │
              └────────────┬────────────┘
                           │              ╭─S34
              ┌────────────▼────────────┐
              │ SETTING OF THRESHOLD VALUE Cth │
              │      BASED ON Cf          │
              └────────────┬────────────┘
```

CONNECTION OF ULTRASOUND TRANSDUCER UNIT — S31

CONFIRM CONTINUITY — S32

MEASUREMENT OF ELECTROSTATIC CAPACITY VALUE Cf — S33

SETTING OF THRESHOLD VALUE Cth BASED ON Cf — S34

**S2** — IS OUTPUT SWITCH TURNED ON? — N

Y

**S3** — SIMULTANEOUS OUTPUT OF HF SIGNAL AND US DRIVE SIGNAL

**S4** — CONTROL OF HF OUTPUT BY MEASUREMENT OF HF VOLTAGE AND HF CURRENT

**S5** — CONTROL OF US OUTPUT BY MEASUREMENT OF US DRIVE VOLTAGE AND US DRIVE CURRENT

**S6** — CALCULATION OF ELECTROSTATIC CAPACITY VALUE Cm OF HF SIGNAL LINE

**S8** — CONTINUE OUTPUT

**S7** — Cm < Cth? — N

**S9** — IS OUTPUT SWITCH TURNED OFF? — N

Y

**S12** — STOP OUTPUT OF HF SIGNAL AND US DRIVE SIGNAL

**S10** — STOP OUTPUT OF HF SIGNAL AND US DRIVE SIGNAL

**S13** — WIRE BREAKAGE DISPLAY/ DISPLAY OF STOP OF OUTPUT

**S11** — END TREATMENT? — N

Y

END

34

# FIG.12

```
                      ( START )
                          │
                          ▼                    ┌─S51
    ┌─────────────────────────────────────────────┐
    │         SETTING OF THRESHOLD VALUE Zth        │
    └─────────────────────────────────────────────┘
                          │
        ┌─────────────────┤
        │                 ▼                    ┌─S52
    ┌───┼─────────────────────────────────────────┐
    │   │   MEASUREMENT (CALCULATION) OF IMPEDANCE Zm │
    └───┼─────────────────────────────────────────┘
        │                 │
        │                 ▼              ┌─S53
        │            ╱─────────────╲
      N │◄──────────      Zm ≥ Zth?
                     ╲─────────────╱
                          │ Y
                          ▼                    ┌─S54
    ┌─────────────────────────────────────────────┐
    │ DETERMINATION OF OCCURRENCE OF ERROR OF WIRE  │
    │         BREAKAGE, STOP OUTPUT                  │
    └─────────────────────────────────────────────┘
                          │
                          ▼
                      ( END )
```

# FIG.13

START

S61

CHECK CONTINUITY

S62

MEASURE IMPEDANCE Zop IN OPEN STATE

S63

SETTING OF THRESHOLD VALUE Zth (= Zop + Zhmin)

S64

CONNECT TREATMENT INSTRUMENT TO POWER SUPPLY APPARATUS

S65

START OUTPUT

S66

MEASUREMENT OF IMPEDANCE Zm

S67

N

$Zm \geq Zth$?

Y

S68

DETERMINATION OF OCCURRENCE OF ERROR OF WIRE BREAKAGE, STOP OUTPUT

END

# FIG.14

EP 2 796 105 B1

# FIG.15

Legend:
- Z1 (300 pF)
- Z2 (200 pF)

Y-axis: Z (kΩ)
X-axis: FREQUENCY (KHz)

# FIG.16

START

S71
OUTPUT HIGH FREQUENCY SIGNALS OF FREQUENCIES f1 AND f2

S72
MEASURE IMPEDANCE Zm2 AT FREQUENCY f2

S73
$Zm2 \geq Zth2$? — N / Y

S74
DETERMINATION OF OCCURRENCE OF ERROR OF WIRE BREAKAGE, STOP OUTPUT

END

# FIG.17

```
                    ( START )
                        │
        ┌──────────────►│
        │               ▼                        ╭─S81
        │   ┌─────────────────────────────────────┐
        │   │  MEASURE IMPEDANCE Zm1 AT FREQUENCY f1 │
        │   └─────────────────────────────────────┘
        │               │
        │               ▼                     ╭─S82
        │  N      ◇─────────────────◇
        └─────────   Zm1 ≥ Zth1?
                  ◇─────────────────◇
                        │ Y
                        ▼                        ╭─S83
            ┌─────────────────────────────────────┐
            │  SWITCH OF OUTPUT OF HIGH FREQUENCY SIGNAL │
            │  FROM FREQUENCY f1 TO FREQUENCY f2       │
            └─────────────────────────────────────┘
        ┌──────────────►│
        │               ▼                        ╭─S84
        │   ┌─────────────────────────────────────┐
        │   │  MEASURE IMPEDANCE Zm2 AT FREQUENCY f2 │
        │   └─────────────────────────────────────┘
        │               │
        │               ▼                     ╭─S85
        │  N      ◇─────────────────◇
        └─────────   Zm2 ≥ Zth2?
                  ◇─────────────────◇
                        │ Y
                        ▼                        ╭─S86
            ┌─────────────────────────────────────┐
            │ DETERMINATION OF OCCURRENCE OF ERROR OF WIRE BREAKAGE, │
            │                STOP OUTPUT           │
            └─────────────────────────────────────┘
                        │
                        ▼
                    ( END )
```

# FIG.18

```
                    ( START )
                        |
                        | ___S91
        ┌───────────────────────────────┐
        │  SETTING OF THRESHOLD VALUE Zth │
        └───────────────────────────────┘
                        |
                        | ___S92
        ┌───────────────────────────────┐
        │       MEASURE IMPEDANCE Zm     │
        └───────────────────────────────┘
                        |
                   ___S93
              ◇ Zm ≥ Zth? ◇ ──── N ────┐
                        |               |  ___S94
                        | Y       ┌──────────────────────┐
                   ___S95         │   CONTINUE OUTPUT     │
        ┌───────────────────────┐└──────────────────────┘
        │ INCREASE OUTPUT VOLTAGE TO V2 │
        └───────────────────────┘
                        |
                        | ___S96
        ┌───────────────────────────────┐
        │ MEASURE IMPEDANCE Zm (V2) AT V2 │
        └───────────────────────────────┘
                        |
                   ___S97
             ◇ Zm (V2) ≥ Zth2? ◇ ──── N ────┐
                        |                     |
                        | Y                   |
                   ___S101                 ___S98
        ┌───────────────────────┐  ┌───────────────────────┐
        │ DETERMINE THAT WIRE    │  │ DETERMINE THAT WIRE    │
        │ BREAKAGE IS PRESENT    │  │ BREAKAGE IS ABSENT     │
        └───────────────────────┘  └───────────────────────┘
                        |                     |
                   ___S102                 ___S99
        ┌───────────────────────┐  ┌───────────────────────┐
        │      STOP OUTPUT       │  │ RETURN OUTPUT TO       │
        └───────────────────────┘  │ ORIGINAL VALUE         │
                        |           └───────────────────────┘
                        |                     |
                        |                  ___S100
                        |           ┌───────────────────────┐
                        |           │  CONTINUE TREATMENT    │
                        |           └───────────────────────┘
                        |                     |
                    ( END )
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080132887 A

- WO 2011089769 A1